# EUROPÄISCHE PATENTSCHRIFT

(11) **EP 0 442 306 B1**
(45) Veröffentlichungstag und Bekanntmachung des Hinweises auf die Patenterteilung: **12.04.1995**
(21) Anmeldenummer: 91100965.2
(22) Anmeldetag: 25.01.1991
(51) Int. Cl.: C07C 25/24, C09K 19/30, C09K 19/32, C09K 19/34, C07C 13/28, C07D 213/06, C07D 239/26, C07D 319/06, C07C 255/50, C07C 43/225, C07C 69/92

(54) **(4E-Cyclohexyl-3-butenyl)aryl-Derivate**
(4E-cyclohexyl-3-butenyl)phenyl derivatives
Dérivés de (4E-cyclohéxyl)-3-buténylphényle

(30) Priorität: 01.02.1990 CH 326/90
(43) Veröffentlichungstag der Anmeldung: 21.08.1991
(73) Patentinhaber: F. Hoffmann-La Roche AG, 4002 Basel (CH)
(72) Erfinder: Kelly, Stephen, Dr., CH-4313 Möhlin (CH)
(74) Vertreter: Cottong, Norbert A.

(56) Entgegenhaltungen:
- EP-A- 0 344 557
- WO-A-91/03450
- DE-A- 3 509 170
- MOLECULAR CRYSTALS AND LIQUID CRYSTALS, (PROCEEDINGS OF THE 13TH INT. LIQ. CRYST. CONF., Vancouver, 22. - 27. Juli 1990, Teil 3), Band 204, 1991, Seiten27-35, Gordon and Breach Science Publishers S.A., US; S.M. KELLY: "The synthesis and liquid crystal transition temperatures of a broad range of mesogens incorporating four-unit-linking groups I"

## Beschreibung

Die vorliegende Erfindung betrifft neue (4E-Cyclohexyl-3-butenyl)aryl-Derivate, flüssigkristalline Gemische, die solche Verbindungen enthalten sowie die Verwendung für elektro-optische Zwecke.

Flüssige Kristalle werden vor allem als Dielektrika in Anzeigevorrichtungen eingesetzt, da die optischen Eigenschaften solcher Substanzen durch eine angelegte Spannung beeinflusst werden können. Elektro-optische Vorrichtungen auf der Basis von Flüssigkristallen sind dem Fachmann bestens bekannt und können auf verschiedenen Effekten beruhen. Beispiele solcher Vorrichtungen sind Zellen mit dynamischer Streuung, DAP-Zellen (Deformation aufgerichteter Phasen), Gast/Wirt-Zellen, TN-Zellen ("twisted nematic") und STN-Zellen ("super-twisted nematic") mit verdrillt nematischer Struktur, SBE-Zellen ("super-birefringence effect"), Phase-Change-Zellen mit einem cholesterisch-nematischen Phasenübergang und OMI-Zellen ("optical mode interference"). Die gebräuchlichsten Anzeigevorrichtungen beruhen auf dem Schadt-Helfrich-Effekt und besitzen eine verdrillt nematische Struktur.
In Appl. Phys. Lett. 36, 899 (1980) und in Recent Developments in Condensed Matter Physics 4, 309 (1981) werden ferner elektro-optische Vorrichtungen auf der Basis von chiral getilteten smektischen Flüssigkristallen vorgeschlagen. Hierbei werden die ferroelektrischen Eigenschaften dieser Materialien ausgenutzt. Als getiltete smektische Phasen eignen sich beispielsweise smektisch C, F, G, H, I und K Phasen. Bevorzugt sind im allgemeinen smektisch C Phasen, welche besonders grosse Ansprechgeschwindigkeiten ermöglichen. Die chiral getilteten Phasen werden üblicherweise mit S_{C}^{*}, S_{F}^{*} etc. bezeichnet, wobei der Stern die Chiralität angibt.

Die Flüssigkristallmaterialien müssen eine gute chemische und thermische Stabilität und eine hohe Stabilität gegenüber elektrischen Feldern und elektromagnetischer Strahlung besitzen. Ferner sollten die Flüssigkristallmaterialien niedere Viskosität aufweisen und in den Zellen kurze Ansprechzeiten, tiefe Schwellenspannungen und einen hohen Kontrast ergeben. Weiterhin sollen sie bei üblichen Betriebstemperaturen eine geeignete Mesophase besitzen, beispielsweise eine nematische, cholesterische oder chiral getiltete smektische Phase. Weitere Eigenschaften, wie die elektrische Leitfähigkeit, die dielektrische Anisotropie und die optische Anisotropie, müssen je nach Zellentyp und Anwendungsgebiet unterschiedlichen Anforderungen genügen. Beispielsweise sollten Materialien für Zellen mit verdrillt nematischer Struktur eine positive dielektrische Anisotropie und eine möglichst geringe elektrische Leitfähigkeit aufweisen. Neben dem generellen Interesse an Flüssigkristallmaterialien mit hoher optischer Anisotropie besteht in letzter Zeit ein vermehrtes Interesse an Materialien mit niedriger optischer Anisotropie, insbesondere für aktiv adressierte Flüssigkristallanzeigen, z.B. bei TFT-Anwendungen (Dünnfilmtransistor) in Fernsehgeräten. Anderseits sollten chiral getiltete smektische Flüssigkristalle eine ausreichend hohe spontane Polarisation besitzen.

Flüssigkristalle werden zwecks Optimierung der Eigenschaften in der Regel als Mischungen mehrerer Komponenten verwendet. Es ist daher wichtig, dass die Komponenten untereinander gut mischbar sind. Cholesterische Gemische können vorzugsweise aus einem oder mehreren optisch aktiven Dotierstoffen und einem nematischen Flüssigkristallmaterial bestehen, und ferroelektrische Flüssigkristalle können vorzugsweise aus einem oder mehreren optisch aktiven Dotierstoffen und einem Flüssigkristallmaterial mit getilteter smektischer Phase bestehen.

Gegenstand der vorliegenden Erfindung sind die Verbindungen der allgemeinen Formel 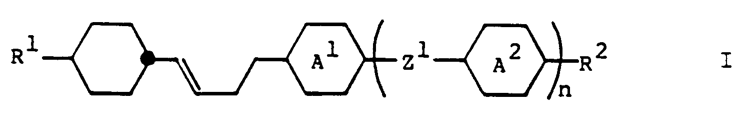
worin n für die Zahl O oder 1 steht; R¹ eine Gruppe R³ oder R³-A³-Z²- und R² eine Gruppe R⁴ oder R⁴-A⁴-Z³- bezeichnen; Ring A¹ unsubstituiertes oder mit Halogen, Cyano und/oder Methyl substituiertes 1,4-Phenylen bedeutet, in welchem gegebenenfalls 1 oder 2 CH-Gruppen durch Stickstoff ersetzt sind; A³, A⁴ und Ring A² unabhängig voneinander unsubstituiertes oder mit Halogen, Cyano und/oder Methyl substituiertes 1,4-Phenylen, in welchem gegebenenfalls 1 oder 2 CH-Gruppen durch Stickstoff ersetzt sind, oder trans-1,4-Cyclohexylen, trans-1,3-Dioxan-2,5-diyl, 1-Cyano-trans-1,4-cyclohexylen, Bicyclo[2.2.2]octan-1,4-diyl, Naphthalin-2,6-diyl, Tetralin-2,6-diyl oder trans-Decalin-2,6-diyl darstellen; Z¹, Z² und Z³ unabhängig voneinander eine einfache Kovalenzbindung, -COO-, -OOC-, -CH₂O-, -OCH₂-, -CH₂CH₂-, -C≡C-, -(CH₂)₃O-, -O(CH₂)₃-, -(CH₂)₄- oder die trans-Form von -CH=CH-CH₂CH₂-, -CH₂CH₂-CH=CH-, -CH=CH-CH₂O- oder -OCH₂-CH=CH- bedeuten; R³ und R⁴ unabhängig voneinander Halogen, Cyano, -NCS, -CF₃, -OCF₃ oder Alkyl bezeichnen, in welchem gegebenenfalls eine >CH-CH< durch >C=C< ersetzt ist und/oder gegebenenfalls eine oder zwei nicht benachbarte Methylengruppen durch -O-, -COO- und/oder -OOC- ersetzt sind und/oder gegebenenfalls eine Methylengruppe durch -CHX- ersetzt ist; und X Halogen, Cyano oder Methyl bedeutet,
mit Ausnahme von 1,2,6-Trifluor-4-[4E-(trans-4-pentylcyclohexyl) -3-butenyl]benzol.

Es wurde überraschenderweise gefunden, dass die Verbindungen der Formel I, welche eine Buten-Brückengruppe zwischen einem Cyclohexanring und einem aromatischen Ring A¹ aufweisen, trotz der hohen Flexibilität der Brückengruppe eine ausgeprägte Tendenz zur Ausbildung von flüssigkristallinen Phasen besitzen. Die optisch inaktiven Verbindungen der Formel I besitzen meist eine nematische, eine smektisch A und/oder eine getiltete smektische (vor allem S_{C}) Phase, und die optisch aktiven Verbindungen der Formel I besitzen meist eine cholesterische, eine smektisch A und/oder eine chiral getiltete smektische (vor allem S_{C}*) Phase. Diese Mesophasentypen sind besonders geeignet zur Erziehung von nematischen, cholesterischen oder chiral getilteten smektischen Phasen in Flüssigkristallgemischen. Verbindungen der Formel I, welche eine höher geordnete smektische Phase, z.B. eine smektisch B Phase aufweisen, sind jedoch ebenfalls gut mischbar mit üblichen Flüssigkristallmaterialien. Die vorliegende Erfindung bietet somit eine breite Palette neuer Komponenten und Mischungen zur weiteren Optimierung und Modifizierung von Flüssigkristallmaterialien.

Die Verbindungen der Formel I besitzen eine hohe chemische Stabilität und eine hohe Stabilität gegenüber elektrischen und magnetischen Feldern. Sie sind farblos, leicht herstellbar und gut löslich untereinander und in bekannten Flüssigkristallmaterialien. Ferner besitzen sie tiefe Viskositäten und ergeben in Anzeigevorrichtungen kurze Ansprechzeiten.

Die Eigenschaften der Verbindungen der Formel I können je nach Zahl und Bedeutung der Ringe und der Substituenten in breiten Bereichen variiert werden. Beispielsweise führen aromatische Ringe zu höheren und gesättigte Ringe zu tieferen Werten der optischen Anisotropie. Eine Erhöhung des Klärpunktes kann beispielsweise durch Einführung eines oder mehrerer weiterer Ringe erreicht werden. Polare Endgruppen, wie Cyano, Halogen, -NCS, -CF₃ oder -OCF₃, und Ringe wie Pyrimidin-2,5-diyl, trans-1,3-Dioxan-2,5-diyl etc. erhöhen die dielektrische Anisotropie, Ringe wie Pyridazin-3,6-diyl, 1-Cyano-trans-1,4-cyclohexylen, 2,3-Dicyano-1,4-phenylen etc. vermindern die dielektrische Anisotropie und laterale Halogen- und Cyanosubstituenten ergeben eine Beitrag zur Dielektrizitätskonstante sowohl parallel als auch senkrecht zur Moleküllängsachse, was je nach Substitutionsmuster zur Erhöhung oder Verminderung der dielektrischen Anisotropie ausgenützt werden kann. Ferner können durch laterale Substituenten an einem oder mehreren Ringen die Mesophasebereiche modifiziert, eine allfällige Tendenz zur Ausbildung hochgeordneter smektischer Phasen weitgehend unterdrückt und oft auch die Löslichkeit verbessert werden. Weiterhin können durch eine C=C-Doppelbindung in der Seitenkette die elastischen Eigenschaften, die Schwellenspannungen, die Ansprechzeiten, die Mesophasen etc. weiter modifiziert werden.

Die erfindungsgemässen Verbindungen ermöglichen daher eine weitere Optimierung der Flüssigkristallgemische und eine Modifizierung der elektro-optischen Eigenschaften in einem weiten Bereich je nach gewünschten Eigenschaften.

Der Ausdruck "Halogen" bezeichnet im Rahmen der vorliegenden Erfindung Fluor, Chlor, Brom oder Jod, insbesondere Fluor oder Chlor.

Der Ausdruck "unsubstituiertes oder mit Halogen, Cyano und/oder Methyl substituiertes 1,4-Phenylen, in welchem gegebenenfalls 1 oder 2 CH-Gruppen durch Stickstoff ersetzt sind," umfasst Gruppen wie 1,4-Phenylen, 2-Fluor-1,4-phenylen, 2,3-Difluor-1,4-phenylen, 2-Chlor-1,4-phenylen, 2-Brom-1,4-phenylen, 2-Cyano-1,4-phenylen, 2,3-Dicyano-1,4-phenylen, 2-Methyl-1,4-phenylen, Pyridin-2,5-diyl, Pyrazin-2,5-diyl, Pyrimidin-2,5-diyl, Pyridazin-3,6-diyl und dergleichen. Bevorzugte Gruppen sind 1,4-Phenylen, 2-Fluor-1,4-phenylen, 2,3-Difluor-1,4-phenylen, Pyridin-2,5-diyl, Pyrazin-2,5-diyl und Pyrimidin-2,5-diyl.

Der Ausdruck "Tetralin-2,6-diyl" bezeichnet 1,2,3,4-Tetrahydronaphthalin-2,6-diyl. Der Ausdruck "trans-Decalin-2,6-diyl" umfasst von trans-Decahydronaphthalin abgeleitete, 2,6-disubstituierte Gruppen, insbesondere (4aαH,8aβH)-Decahydronaphthalin-2α,6β-diyl.

Der Ausdruck "Alkyl, in welchem gegebenenfalls eine >CH-CH< durch >C=C< ersetzt ist und/oder gegebenenfalls eine oder zwei nicht benachbarte Methylengruppen durch -O-, -COO- und/oder -OOC- ersetzt sind und/oder gegebenenfalls eine Methylengruppe durch -CHX- ersetzt ist," umfasst geradkettige und verzweigte (gegebenenfalls chirale) Reste wie Alkyl, 1E-Alkenyl, 3E-Alkenyl, 4-Alkenyl, Alkenyl mit endständiger Doppelbindung, Alkoxy, 2E-Alkenyloxy, 3-Alkenyloxy, Alkenyloxy mit endständiger Doppelbindung, Alkoxyalkyl, Alkenyloxyalkyl, Alkoxycarbonyl, Alkoxycarbonylalkoxy, Alkanoyloxy, 1-Haloalkyl, 2-Haloalkyl, 2-Haloalkoxy, 2-Haloalkoxycarbonyl, 1-Cyanoalkyl, 2-Cyanoalkyl, 2-Cyanoalkoxy, 2-Cyanoalkoxycarbonyl, 1-Methylalkyl, 2-Methylalkyl, 1-Methylalkoxy, 2-Methylalkoxy, 2-Methylalkoxycarbonyl und dergleichen. Beispiele bevorzugter Reste sind Methyl, Aethyl, Propyl, Butyl, Pentyl, Hexyl, Heptyl, Octyl, Nonyl, Decyl, Undecyl, Dodecyl, 1-Methylpropyl, 1-Methylheptyl, 2-Methylbutyl, 3-Methylpentyl, Vinyl, 1E-Propenyl, 1E-Butenyl, 1E-Pentenyl, 1E-Hexenyl, 1E-Heptenyl, 3-Butenyl, 3E-Pentenyl, 3E-Hexenyl, 3E-Heptenyl, 4-Pentenyl, 4Z-Hexenyl, 4Z-Heptenyl, 5-Hexenyl, 6-Heptenyl, 7-Octenyl, 8-Nonenyl, 9-Decenyl, 10-Undecenyl, 11-Dodecenyl, Methoxy, Aethoxy, Propyloxy, Butyloxy, Pentyloxy, Hexyloxy, Heptyloxy, Octyloxy, Nonyloxy, Decyloxy, Undecyloxy, Dodecyloxy, 1-Methylpropyloxy, 1-Methylheptyloxy, 2-Methylbutyloxy, 3-Methylpentyloxy, Allyloxy, 2E-Butenyloxy, 2E-Pentenyloxy, 2E-Hexenyloxy, 2E-Heptenyloxy, 3-Butenyloxy, 3Z-Pentenyloxy, 3Z-Hexenyloxy, 3Z-Heptenyloxy, 4-Pentenyloxy, 5-Hexenyloxy, 6-Heptenyloxy, 7-Octenyloxy, 8-Nonenyloxy, 9-Decenyloxy, 10-Undecenyloxy, 11-Dodecenyloxy, Methoxymethyl, Aethoxymethyl, Propyloxymethyl, Allyloxymethyl, Methoxycarbonyl, Aethoxycarbonyl, Propyloxycarbonyl, 1-Methylpropyloxycarbonyl, 1-(Methoxycarbonyl)äthoxy, 1-(Aethoxycarbonyl)äthoxy, Acetoxy, Propionyloxy, Butyryloxy, 1-Fluorpropyl, 1-Fluorpentyl, 1-Chlorpropyl, 2-Fluorpropyl, 2-Fluorpentyl, 2-Chlorpropyl, 2-Fluorpropyloxy, 2-Fluorbutyloxy, 2-Fluorpentyloxy, 2-Fluorhexyloxy, 2-Chlorpropyloxy, 2-Fluorbutyloxy, 2-Fluorpropyloxycarbonyl, 2-Fluorbutyloxycarbonyl, 2-Fluorpentyloxycarbonyl, 2-Fluor-3-methylbutyloxycarbonyl, 2-Fluor-4-methylpentyloxycarbonyl, 2-Chlorpropyloxycarbonyl, 1-Cyanopropyl, 1-Cyanopentyl, 2-Cyanopropyl, 2-Cyanopentyl, 2-Cyanopropyloxy, 2-Cyanobutyloxy, 2-Cyanopentyloxy, 2-Cyanohexyloxy, 2-Cyanopropyloxycarbonyl, 2-Cyanobutyloxycarbonyl, 2-Cyano-3-methylbutyloxycarbonyl, 2-Cyano-4-methylpentylokycarbonyl und dergleichen.

Bevorzugte Verbindungen der Formel I sind die Verbindungen der allgemeinen Formeln 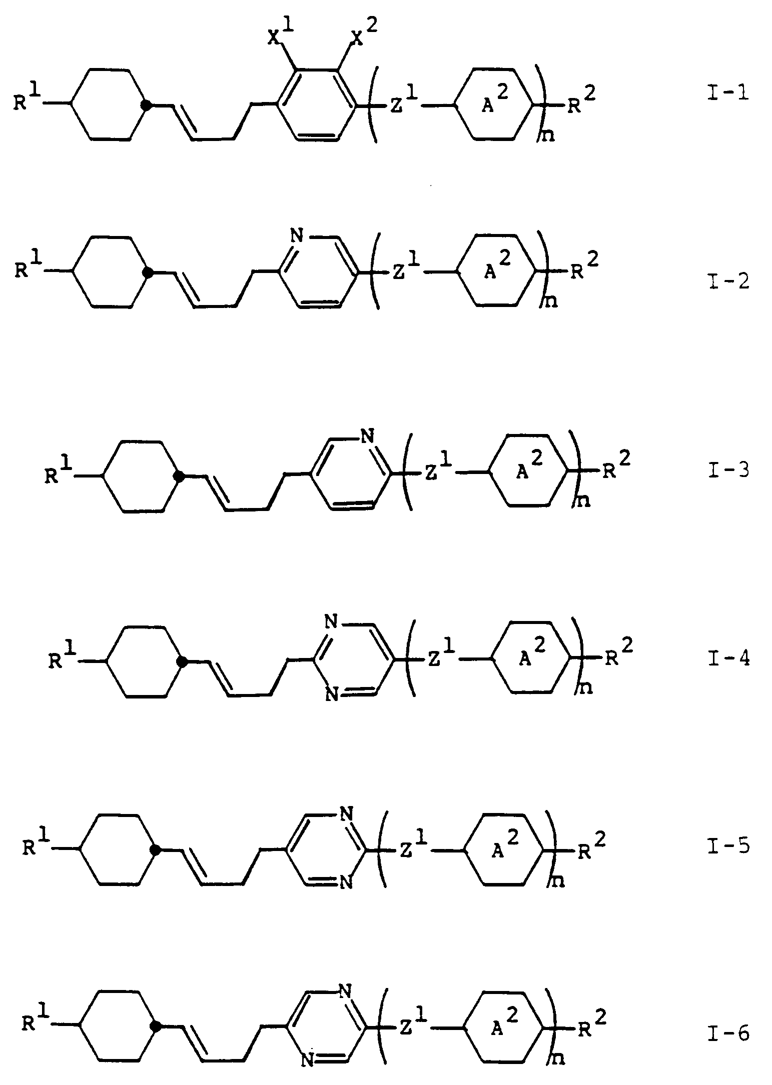
worin n, R¹, R², Z¹ und Ring A² die obigen Bedeutungen haben und X¹ und X² unabhängig voneinander Wasserstoff, Halogen, Cyano oder Methyl bezeichnen.

In den obigen Formeln I und I-1 bis I-6 steht Ring A² vorzugsweise für unsubstituiertes oder mit Halogen, Cyano und/oder Methyl monosubstituiertes oder 2,3-disubstituiertes 1,4-Phenylen, für trans-1,4-Cyclohexylen, trans-1,3-Dioxan-2,5-diyl, 1-Cyano-trans-1,4-cyclohexylen, Bicyclo-[2.2.2]octan-1,4-diyl, Naphthalin-2,6-diyl, Tetralin-2,6-diyl, trans-Decalin-2,6-diyl oder in den Formeln I und I-1 auch für Pyridin-2,5-diyl, Pyrimidin-2,5-diyl oder Pyrazin-2,5-diyl.

A³ und A⁴ stehen unabhängig voneinander bevorzugt für trans-1,4-Cyclohexylen oder für unsubstituiertes oder mit Halogen, Cyano und/oder Methyl substituiertes 1,4-Phenylen, insbesondere für trans-1,4-Cyclohexylen.

Vorzugsweise stehen Z¹, Z² und Z³ je für eine einfache Kovalenzbindung oder eine der Gruppen Z¹, Z² und Z³ (bevorzugt Z¹) auch für -COO-, -OOC-, -CH₂O-, -OCH₂-, -CH₂CH₂-, -C≡C-, -(CH₂)₃O-, -O(CH₂)₃-, -(CH₂)₄- oder die trans-Form von -CH=CH-CH₂CH₂-, -CH₂CH₂-CH=CH-, -CH=CH-CH₂O- oder -OCH₂-CH=CH-.

Bevorzugte Basismaterialien für flüssigkristalline Gemische sind die bicyclischen und die tricyclischen Verbindungen der Formeln I und I-1 bis I-6. In den bicyclischen Verbindungen steht n für die Zahl 0, R¹ für R³ und R² für R⁴. In den tricyclischen Verbindungen steht n für die Zahl 1, R¹ für R³ und R² für R⁴ oder n für die Zahl O, R¹ für R³-A³-Z²- und R² für R⁴ oder n für die Zahl O, R¹ für R³ und R² für R⁴-A⁴-Z³-. Verbindungen mit 4 oder 5 Ringen sind jedoch dann bevorzugt, wenn hohe Klärpunkte erwünscht sind (z.B. zur Verwendung als Dotierstoff zur Klärpunktserhöhung oder als stationäre Phase in der Gaschromatographie).

Beispiele besonders bevorzugter Untergruppen von Verbindungen der Formel I sind die Verbindungen der allgemeinen Formeln 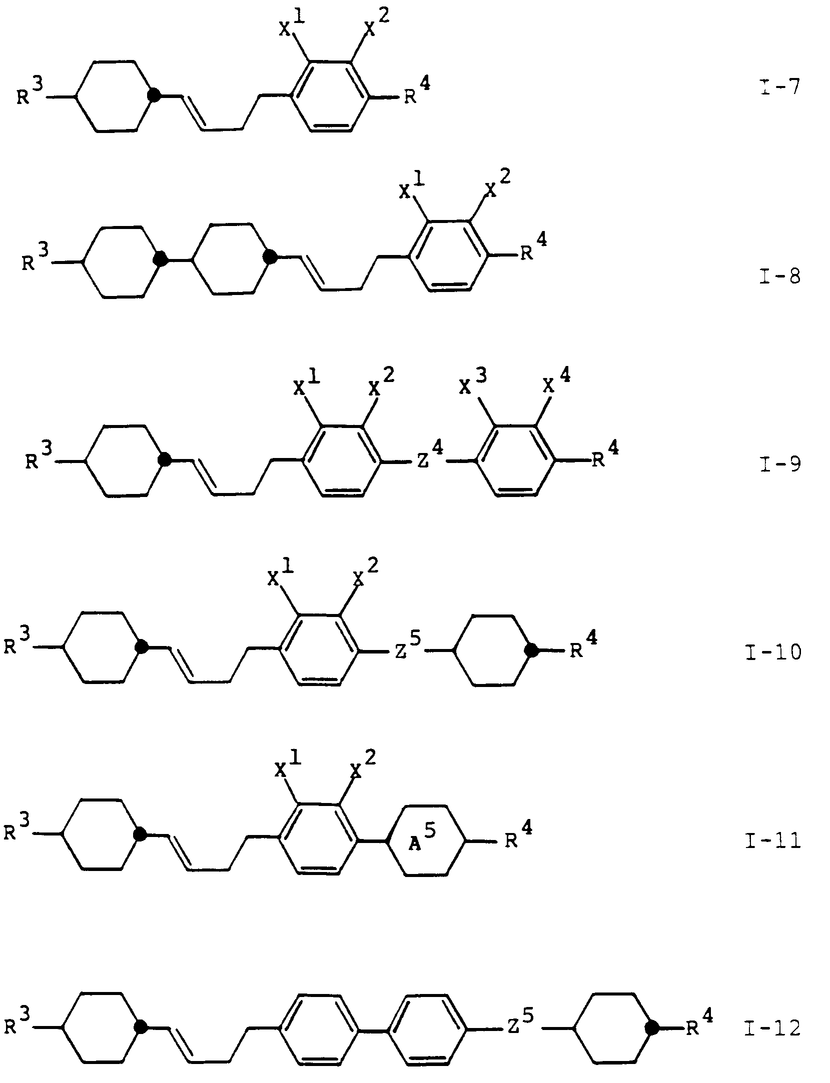 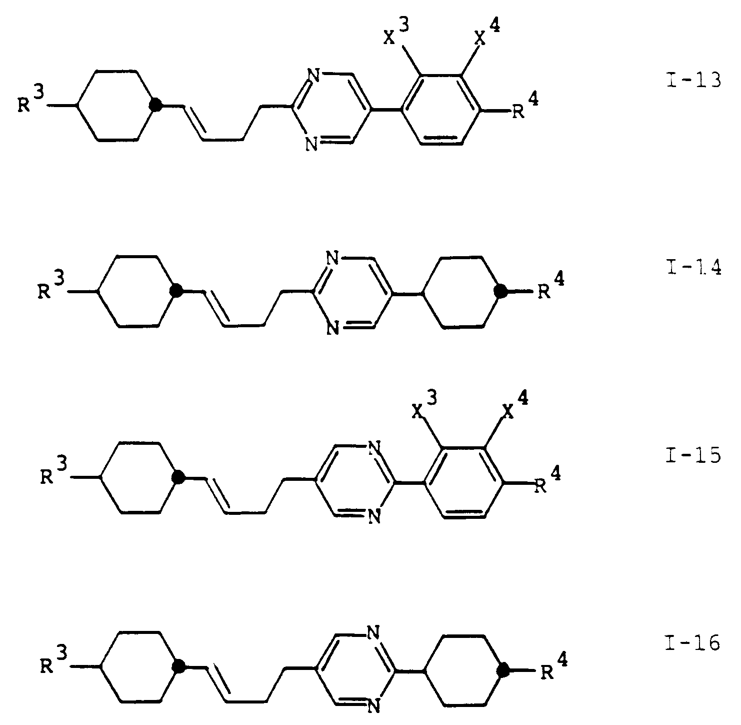
worin R³ und R⁴ die obigen Bedeutungen haben; X¹, X², X³ und X⁴ unabhängig voneinander Wasserstoff, Halogen, Cyano oder Methyl bezeichnen; Z⁴ eine einfache Kovalenzbindung, -COO-, -OOC- oder -C≡C-bedeutet; Z⁵ eine einfache Kovalenzbindung, -OOC-, -OCH₂-, -CH₂CH₂-, -C≡C-, -O(CH₂)₃-, -(CH₂)₄- oder die trans-Form von -CH₂CH₂-CH=CH- oder -OCH₂-CH=CH-bezeichnet; und Ring A⁵ Pyridin-2,5-diyl, Pyrimidin-2,5-diyl, Pyrazin-2,5-diyl, trans-1,3-Dioxan-2,5-diyl, Bicyclo[2.2.2]octan-1,4-diyl, Naphthalin-2,6-diyl, Tetralin-2,6-diyl oder trans-Decalin-2,6-diyl darstellt.
Im allgemeinen sind Verbindungen der Formel I ohne laterale Substituenten oder mit lateralen Fluorsubstituenten (vorzugsweise höchstens einer oder zwei laterale Fluorsubstituenten) bevorzugt, d.h. in obigen Formeln I-1, I-7 bis I-11, I-13 und I-15 stehen die Substituenten X¹, X², X³ und X⁴ vorzugsweise für Wasserstoff oder Fluor, insbesondere für Wasserstoff oder 1 oder 2 der Substituenten X¹, X², X³ und X⁴ auch für Fluor. Es ist jedoch für den Fachmann klar, dass anstelle von Fluor auch andere Halogensubstituenten, insbesondere Chlor, verwendet werden können und dass durch laterale Methyl-Substitution oft die Löslichkeit verbessert werden kann bzw. durch Cyano-Substituenten ein stärkeres laterales Dipolmoment erzeugt werden kann.
In Formel I-11 kann ein gegebenenfalls vorhandener Pyridinring, Pyrimidinring, Pyrazinring oder 1,3-Dioxanring jeweils in 2-Stellung oder in 5-Stellung mit dem Benzolring verknüpft sein.

Vorzugsweise steht höchstens einer der Reste R³ und R⁴ (vorzugsweise R⁴) für Halogen, Cyano, -NCS, -CF₃ oder -OCF₃, d.h. R³ und R⁴ bezeichnen unabhängig voneinander vorzugsweise Alkyl, in welchem gegebenenfalls eine >CH-CH< durch >C=C< ersetzt ist und/oder gegebenenfalls eine oder zwei nicht benachbarte Methylengruppen durch -O-, -COO- und/oder -OOC- ersetzt sind und/oder gegebenenfalls eine Methylengruppe durch -CHX- ersetzt ist, oder einer der Reste R³ und R⁴ (vorzugsweise R⁴) bezeichnet auch Halogen, Cyano, -NCS, -CF₃ oder -OCF₃. Ein endständiger Halogenrest und die Gruppe -NCS stehen vorzugsweise an einem aromatischen Ring, insbesondere einem Benzol-, Pyridin-, Pyrimidin- oder Pyrazinring.

R³ und R⁴ in den obigen Formeln I und I-1 bis I-16 besitzen vorzugsweise höchstens je etwa 18, insbesondere höchstens je etwa 12 Kohlenstoffatome.

Für nematische und cholesterische Anwendungen sind im allgemeinen kurze Reste R³ und R⁴ (z.B. Reste mit höchstens 12, vorzugsweise höchstens 7 Kohlenstoffatomen) bevorzugt und vorzugsweise kann auch einer der Reste Halogen, Cyano, -NCS, -CF₃ oder -OCF₃ bedeuten. Für smektische Anwendungen (insbesondere getiltete smektische Phasen) sind im allgemeinen diejenigen Verbindungen bevorzugt, worin R³ und R⁴ unabhängig voneinander Alkyl bezeichnen, in welchem gegebenenfalls eine >CH-CH< durch >C=C< ersetzt ist und/oder gegebenenfalls eine oder zwei nicht benachbarte Methylengruppen durch -O-, -COO- und/oder -OOC- ersetzt sind und/oder gegebenenfalls eine Methylengruppe durch -CHX- ersetzt ist, und die Summe der Kohlenstoffatome in R³ und R⁴ zusammen mindestens 10, vorzugsweise mindestens 12 beträgt.

Bevorzugte Reste R³ sind Alkyl, Alkenyl, Alkoxy, Alkenyloxy, Alkoxycarbonyl, Alkenyloxycarbonyl, Alkanoyloxy und Alkenoyloxy, insbesondere Alkyl und Alkenyl. Besonders bevorzugt sind im allgemeinen Reste R³ mit bis zu 12 Kohlenstoffatomen. Bevorzugte Reste R⁴ sind Alkyl, Alkenyl, Alkoxy, Alkenyloxy, Alkoxycarbonyl, Alkenyloxycarbonyl, Alkanoyloxy und Alkenoyloxy (insbesondere Alkyl, Alkenyl, Alkoxy und Alkenyloxy) sowie Halogen (insbesondere Fluor und Chlor), Cyano, -NCS, -CF₃ und -OCF₃. Besonders bevorzugt sind im allgemeinen Reste R⁴ mit bis zu 12 Kohlenstoffatomen.

Geradkettige Reste R³ bzw. R⁴ sind im allgemeinen bevorzugt. Um beispielsweise chirale Dotierstoffe für cholesterische oder für chiral getiltete smektische Flüssigkristalle zu erhalten, können jedoch vorzugsweise auch einer oder beide Reste R³ und R⁴ verzweigt-kettig chiral sein und/oder eine Gruppe -CHX-, worin X Halogen (vorzugsweise Fluor oder Chlor), Cyano oder Methyl bedeutet, anstelle einer Methylengruppe aufweisen. Um für chiral getiltete smektische Anwendungen eine hohe spontane Polarisation zu erhalten, sollte das Chiralitätszentrum (d.h. die Kettenverzweigung oder der Halogen-, Methyl- oder Cyanosubstituent) vorzugsweise nahe am Ringsystem, beispielsweise in 1- oder 2-Stellung des Restes R³ bzw. R⁴ sein. Ferner bleibt die Tendenz zur Bildung flüssigkristalliner Phasen grundsätzlich erhalten, wenn 1 oder 2 nicht benachbarte Methylengruppen durch -O-, -COO- und/oder -OOC- ersetzt werden, was u.a. zur Herstellung chiraler Reste aus natürlichen, optisch aktiven Säuren, Alkoholen etc. ausgenutzt werden kann (z.B. 2-Alkoxycarbonyläthoxy aus Milchsäure).

Die Mesophasenbereiche, die Schwellenspannung, die Ansprechgeschwindigkeit, die Steilheit der Transmissionskennlinie etc. können ferner variiert werden durch Wahl der Stellung der C=C-Doppelbindung in ungesättigten Resten wie Alkenyl, Alkenyloxy und dergleichen. Der Effekt ist grundsätzlich bekannt z.B. aus Mol. Cryst. Liq. Cryst. 122, 241 (1985), 131, 109 (1985) und 148, 123 (1987). Bevorzugt sind Reste, welche die Doppelbindung in 1-Stellung (insbesondere E-Isomer), in 3-Stellung (insbesondere E-Isomer) oder in 4-Stellung (insbesondere Z-Isomer) der Kette unter Einschluss allfälliger Heteroatome aufweisen, wie 1E-Alkenyl, 3E-Alkenyl, 4Z-Alkenyl, 2E-Alkenyloxy, 3Z-Alkenyloxy und dergleichen. Ferner kann die Doppelbindung vorzugsweise auch in endständiger Position stehen, insbesondere im Falle von Verbindungen für smektische Anwendungen. Beispiele bevorzugter Reste mit endständiger Stellung der Doppelbindung sind 6-Heptenyl, 7-Octenyl, 8-Nonenyl, 9-Decenyl, 10-Undecenyl, 11-Dodecenyl, 5-Hexenyloxy, 6-Heptenyloxy, 7-Octenyloxy, 8-Nonenyloxy, 9-Decenyloxy, 10-Undecenyloxy, 11-Dodecenyloxy und dergleichen.

Die Herstellung der Verbindungen der Formel I kann in an sich bekannter Weise erfolgen. Bevorzugte Methoden werden anhand der folgenden Schemata 1-4 veranschaulicht, worin R¹, R², Z¹, n und die Ringe A¹ und A² die obigen Bedeutungen haben, -O-THP Tetrahydro-2-pyranyloxy bezeichnet und R⁵ eine Alkylgruppe bedeutet, in welcher gegebenenfalls eine >CH-CH< durch >C=C< ersetzt ist und/oder gegebenenfalls eine nicht in 1-Stellung stehenden Methylengruppe durch -O-, -COO- oder -OOC- ersetzt ist und/oder gegebenenfalls eine Methylengruppe durch -CHX-ersetzt (worin X die obige Bedeutung hat). 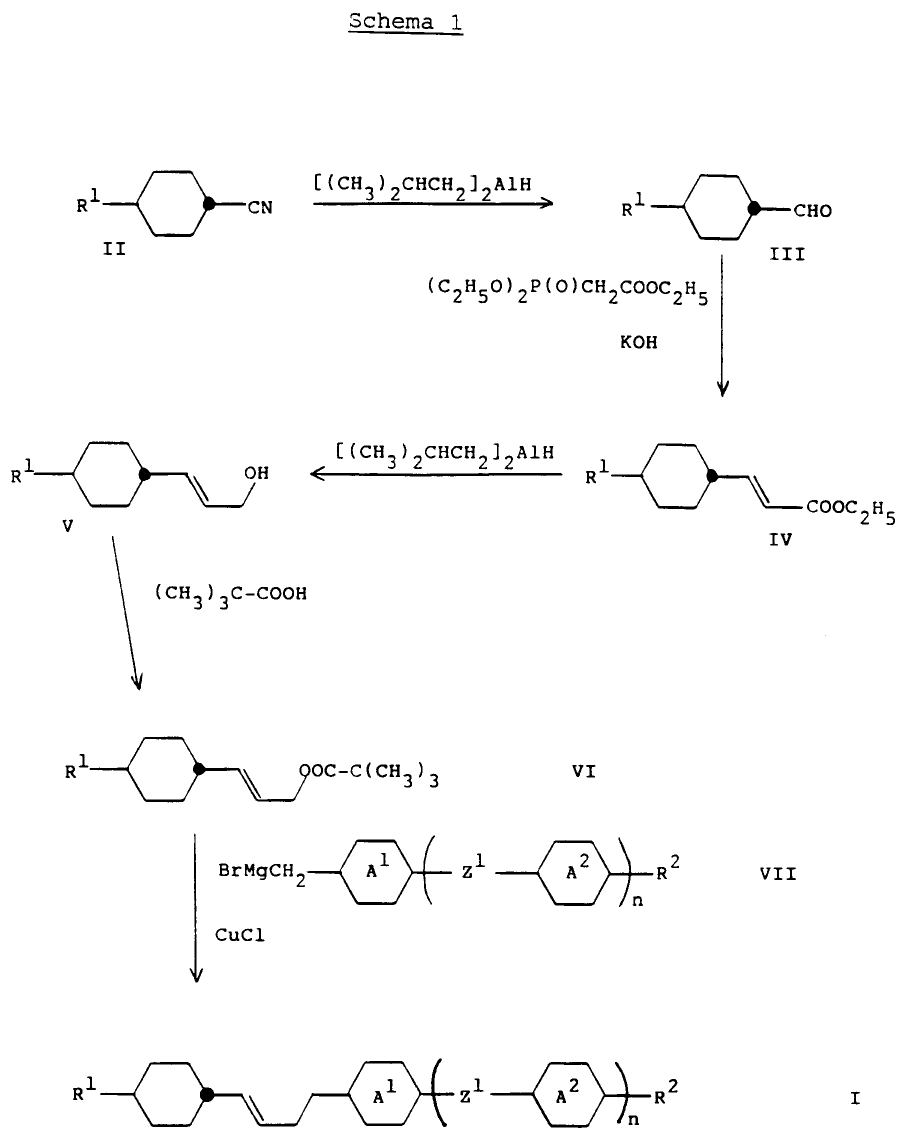 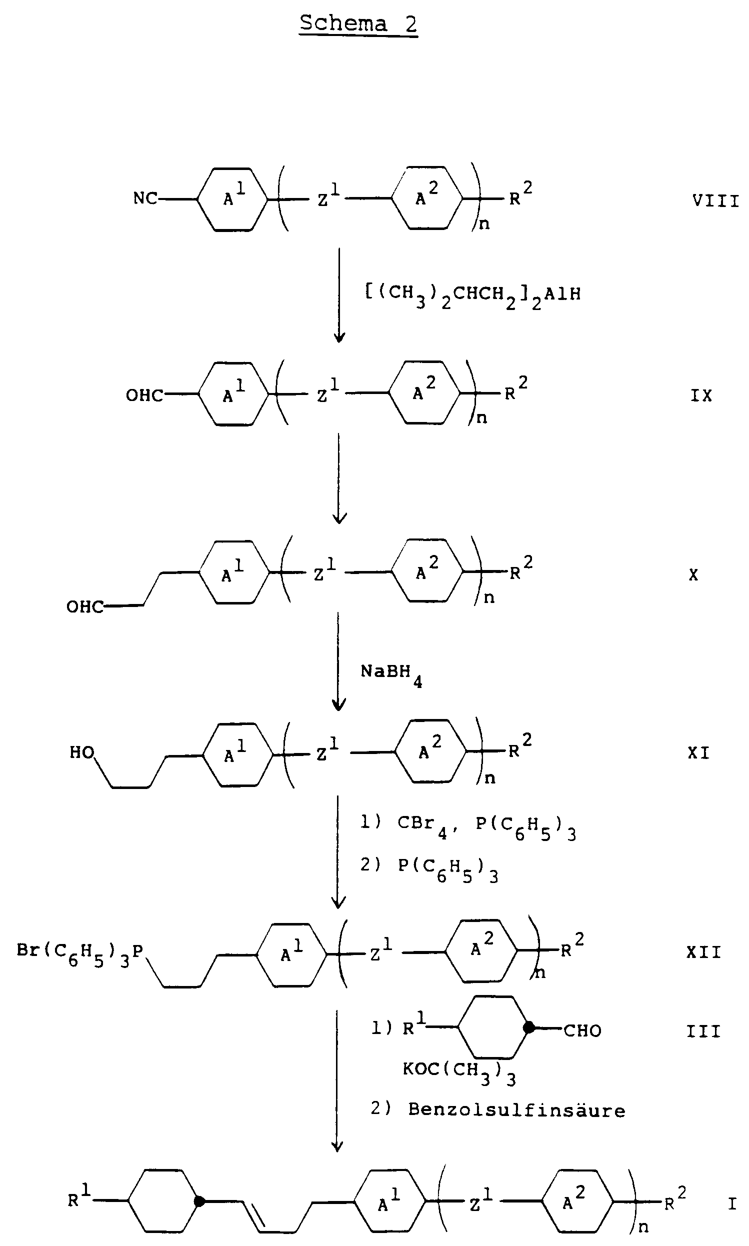 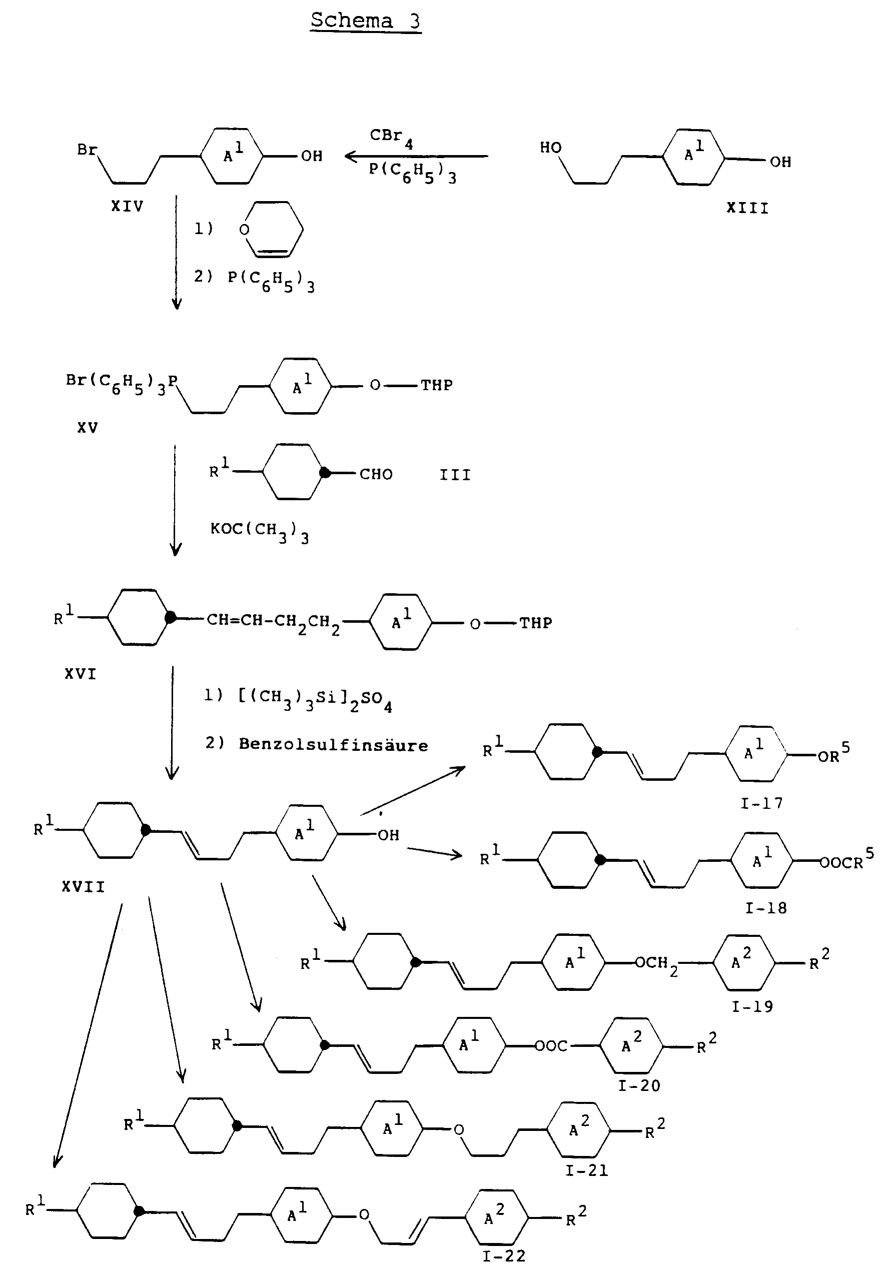 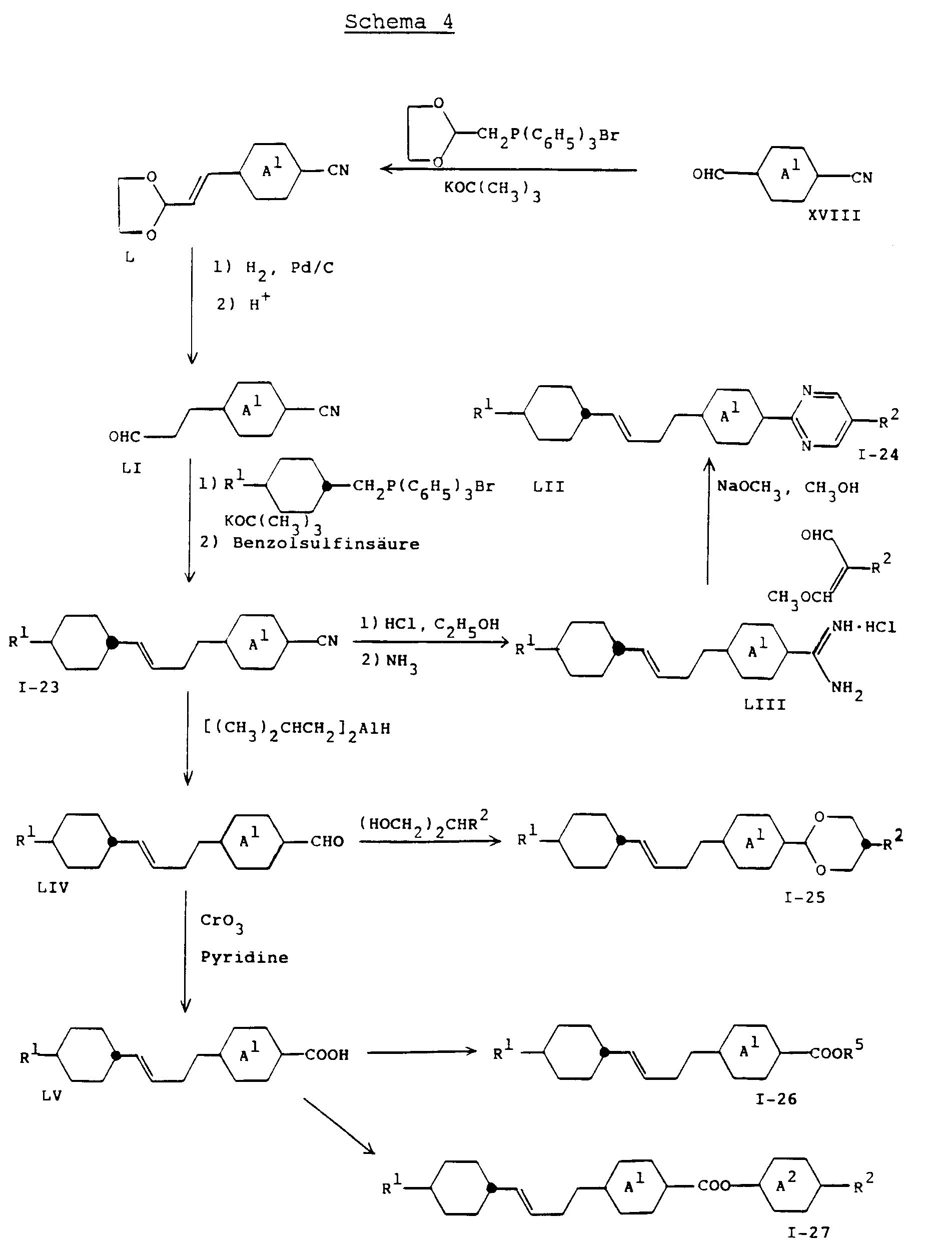
Die Ausgangsmaterialien der Formeln II, III, VIII, IX X, XIII und XVIII sind bekannte oder Analoge bekannter Verbindungen und können nach bekannten Methoden hergestellt werden. Solche Verbindungen wurden bereits als Flüssigkristalle oder Zwischenprodukte für Flüssigkristalle beschrieben. Die Herstellung der Grignard-Reagenzien der Formel VII und der Phosphoniumsalze der Formel LII aus den entsprechenden Bromiden ist dem Fachmann ebenfalls geläufig.

Alternativ zu der in Schema 2 angegebenen Methode kann ferner - wie in Schema 4 illustriert - der Propionaldehyd der Formel X durch Wittig-Reaktion mit dem Phosphoniumsalz der Formel LII umgesetzt und das erhaltene cis/trans-Gemisch mit Benzolsulfinsäure zur Verbindung der Formel I isomerisiert werden.

Die in den Schemata 1 und 2 dargestellten Methoden können sinngemäss auch zur Herstellung von Zwischenprodukten der Verbindungen der Formel I dienen, welche anstelle von R¹, Z¹, Ring A² und/oder R² eine geeignete funktionelle Gruppe aufweisen. Die erhaltenen Zwischenprodukte mit der Buten-Brückengruppe können anschliessend in an sich bekannter Weise in Verbindungen der Formel I überführt werden. Insbesondere erfolgt die Veresterung zu Verbindungen der Formel I, welche eine oder mehrere Estergruppen aufweisen, mit Vorteil erst nach der Bildung der Buten-Brückengruppen. Selbstverständlich können aber auch andere Gruppen, wie z.B. Alkenylreste, Aetherreste, heterocyclische Ringe erst nachträglich gebildet werden. Beispiele derartiger Methoden sind in den Schemata 3 und 4 illustriert.

Die Veresterung der Verbindung der Formel V kann in an sich bekannter Weise erfolgen, z.B. mit Pivalinsäure in Gegenwart von 4-(Dimethylamino)pyridin und N,N'-Dicyclohexylcarbodiimid oder mit Pivalinsäurechlorid in Gegenwart von Pyridin.

Die Umsetzung des Aldehyds der Formel IX zum homologen Aldehyd der Formel X kann nach üblichen Methoden erfolgen. Beispielsweise kann sukzessive je eine Methylengruppe eingeführt werden durch Wittig-Reaktion des Aldehyds mit Methoxmethyl-triphenylphosphoniumchlorid und anschliessende Hydrolyse des erhaltenen Enoläthers (z.B mit verdünnter Salzsäure). Direkte Kettenverlängerung um zwei Methylengruppen kann nach der in Schema 4 illustrierten Methode erreicht werden, wenn keine leicht hydrierbaren Gruppen im Molekül vorhanden sind.

Nach obiger Methode können Aldehyde in homologe Aldehyde mit grundsätzlich beliebig verlängerter Kette übergeführt werden, was zur Herstellung verschiedenster Derivate ausgenützt werden kann. Beispielsweise kann der Aldehyd der Formel LIV, gegebenenfalls nach Uberführung in den geeigneten homologen Aldehyd, durch Wittig-Reaktion in ein Alkenylderivat oder eine Verbindung mit einer weiteren Buten-Brückengruppe übergeführt werden oder durch Umsetzung mit 2-R²-1,3-Propandiol in Dioxanderivate übergeführt werden, in welchem der Dioxanring mit Ring A¹ direkt oder über -(CH₂)₂- oder-(CH₂)₄- verknüpft ist. Durch Reduktion der Aldehyde mit Natriumborhydrid oder durch Oxidation der Aldehyde mit Chromsäure sind auch die homologen Alkohole bzw. Carbonsäuren zugänglich, welche in an sich bekannter Weise zu Verbindungen der Formel I veräthert oder verestert werden können. Beispiele geeigneter Verätherungs- und Veresterungsmöglichkeiten sind in den Schemata 3 und 4 für die Verbindungen der Formeln XVII und LV illustriert.

Weitere Methoden, insbesondere Kettenverlängerungsreaktionen und Methoden zur Einführung von Alkenylsubstituenten, verschiedenen Brückengruppen und heterocyclischen Ringen sind dem Fachmann grundsätzlich bekannt, z.B. aus EP-A-0 122 389, EP-A-0 169 327, EP-A-0 172 360, EP-A-0 167 912, EP-A-0 168 683, EP-A-0 242 716 und EP-A-0 344 557.

Die Verbindungen der Formel I können in Form von Gemischen untereinander und/oder mit andern Flüssigkristallkomponenten verwendet werden. Geeignete Flüssigkristallkomponenten sind dem Fachmann in grosser Zahl bekannt, z.B. aus D. Demus et al., Flüssige Kristalle in Tabellen, VEB Deutscher Verlag für Grundstoffindustrie, Leipzig, Bände I und II, und viele davon sind zudem im Handel erhältlich.

Die Erfindung betrifft daher ebenfalls ein flüssigkristallines Gemisch mit mindestens 2 Komponenten, welches dadurch gekennzeichnet ist, dass mindestens eine Komponente eine Verbindung der Formel I (insbesondere eine der als bevorzugt genannten Verbindungen) ist.

Aufgrund der guten Löslichkeit sowie anderseits der grossen Variationsbreite der Eigenschaften und Anwendungsbereiche, kann der Anteil an Verbindungen der Formel I in den erfindungsgemässen Gemischen in einem breiten Bereich variieren und etwa O,1 bis 1OO Gew.% betragen. Beispielsweise kann das Gemisch aus Verbindungen der Formel I bestehen. Anderseits werden z.B. chirale Dotierstoffe oft nur in relativ kleinen Mengen z.B. etwa O,1 bis 1O Gew.% eingesetzt. Im allgemeinen beträgt jedoch der Anteil an Verbindungen der Formel I in den erfindungsgemässen Gemischen etwa 1 - 60 Gew.%. Bevorzugt ist in der Regel ein Bereich von etwa 5 - 30 Gew.%.

Die erfindungsgemässen Gemische für nematische oder cholesterische Anwendungen enthalten neben einer oder mehreren Verbindungen der Formel I vorzugsweise eine oder mehrere Verbindungen aus der Gruppe der Verbindungen der allgemeinen Formeln 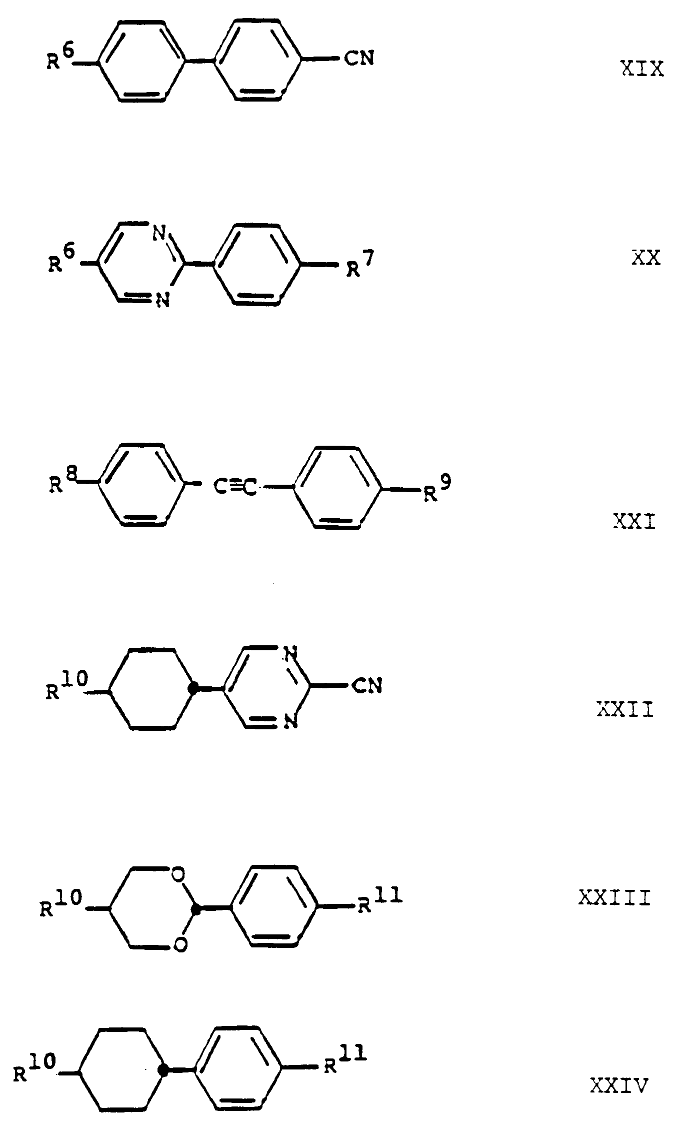 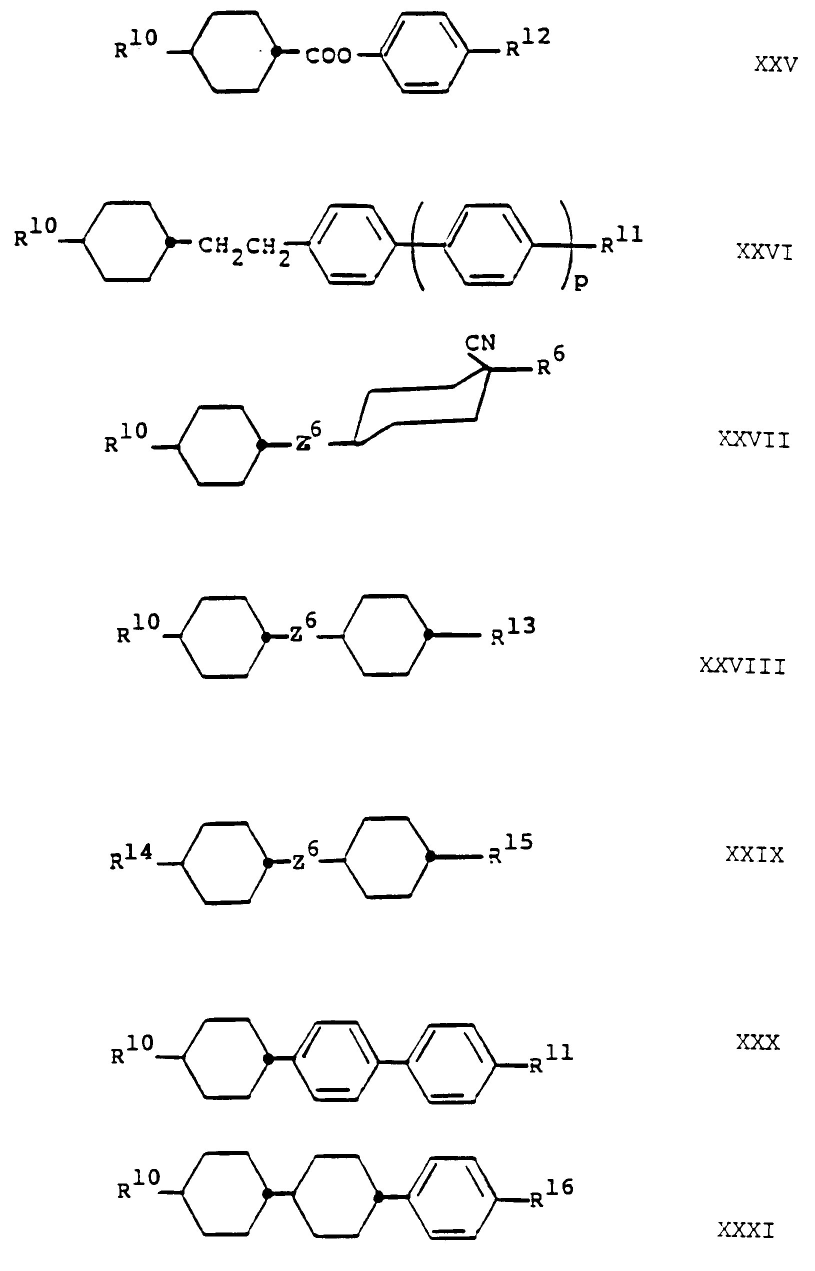 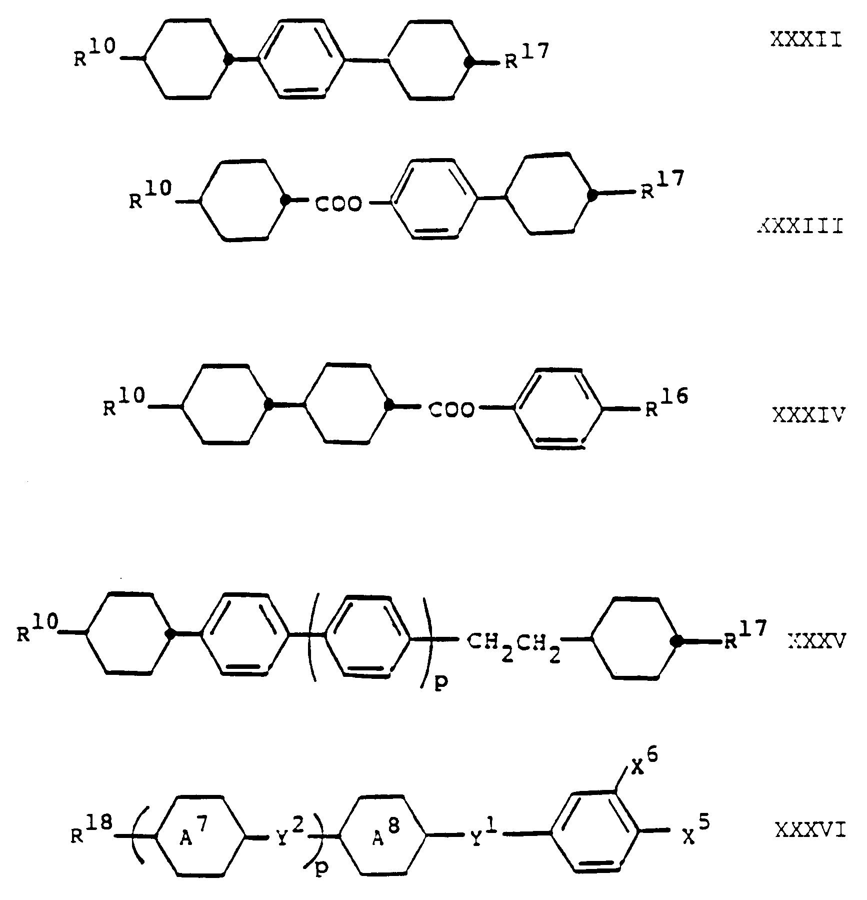
worin R⁶ Alkyl, 3E-Alkenyl oder 4-Alkenyl bedeutet; R⁷ Cyano oder Fluor darstellt; R⁸ und R⁹ Alkyl oder Alkoxy bezeichnen; R¹⁰ un R¹⁷ unabhängig voneinander Alkyl, 1E-Alkenyl, 3E-Alkenyl oder 4-Alkenyl bedeuten; R¹¹ Cyano, -NCS, Alkyl, 3E-Alkenyl, 4-Alkenyl, Alkoxy, 2E-Alkenyloxy oder 3-Alkenyloxy bezeichnet; R¹² Alkoxy, 2E-Alkenyloxy oder 3-Alkenyloxy bedeutet; p für die Zahl O oder 1 steht; Z⁶ eine einfache Kovalenzbindung oder -CH₂CH₂- darstellt; R¹³ Cyano, Alkyl, 1E-Alkenyl, 3E-Alkenyl oder 4-Alkenyl bedeutet; R¹⁴ Alkyl, 1E-Alkenyl, 3E-Alkenyl oder 4-Alkenyl bezeichnet; R¹⁵ Alkoxy, 2E-Alkenyloxy, 3-Alkenyloxy, Alkoxymethyl oder (2-Alkenyl)oxymethyl darstellt; R¹⁶ Cyano, Alkyl, 3E-Alkenyl, 4-Alkenyl, Alkoxy, 2E-Alkenyloxy oder 3-Alkenyloxy bezeichnet; X⁵ Fluor oder Chlor und X⁶ Wasserstoff, Fluor oder Chlor bezeichnen; R¹⁸ Alkyl, 3E-Alkenyl, 4-Alkenyl, Alkoxy, 2E-Alkenyloxy oder 3-Alkenyloxy bedeutet; eine der Gruppen Y¹ und Y² eine einfache Kovalenzbindung, -COO-, -OOC-, -CH₂CH₂-, -CH₂O- oder -OCH₂- und die andere der Gruppen Y¹ und Y² eine einfache Kovalenzbindung bedeutet; und die Ringe A⁷ und A⁸ unabhängig voneinander trans-1,4-Cyclohexylen, in welchem gegebenenfalls 2 nicht benachbarte CH₂-Gruppen durch Sauerstoff ersetzt sind, oder 1,4-Phenylen, in welchem gegebenenfalls 1 oder 2 CH-Gruppen durch Stickstoff ersetzt sind, darstellen.

Vorzugsweise besitzen die Reste R⁶ und R⁸-R¹⁸ höchstens je 12 Kohlenstoffatome, insbesondere höchstens je 7 Kohlenstoffatome. Geradkettige Reste sind im allgemeinen bevorzugt.

Die erfindungsgemässen Gemische für smektische Anwendungen (insbesondere für getiltete smektische oder chiral getiltete smektische Phasen) enthalten neben einer oder mehreren Verbindungen der Formel I vorzugsweise eine oder mehrere Verbindungen aus der Gruppe der Verbindungen der allgemeinen Formeln 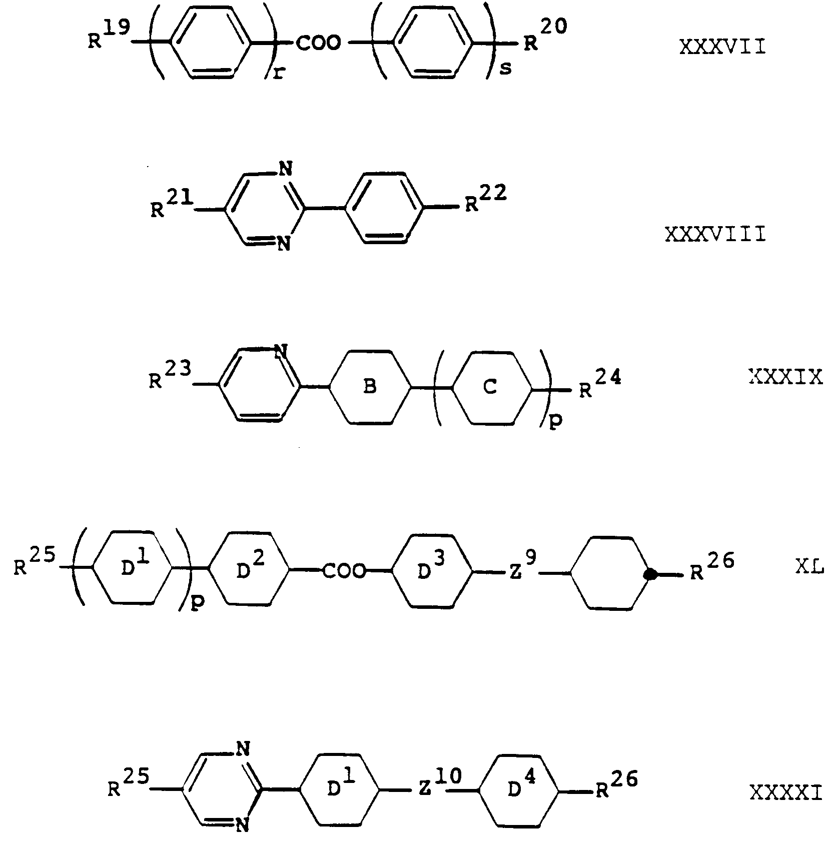 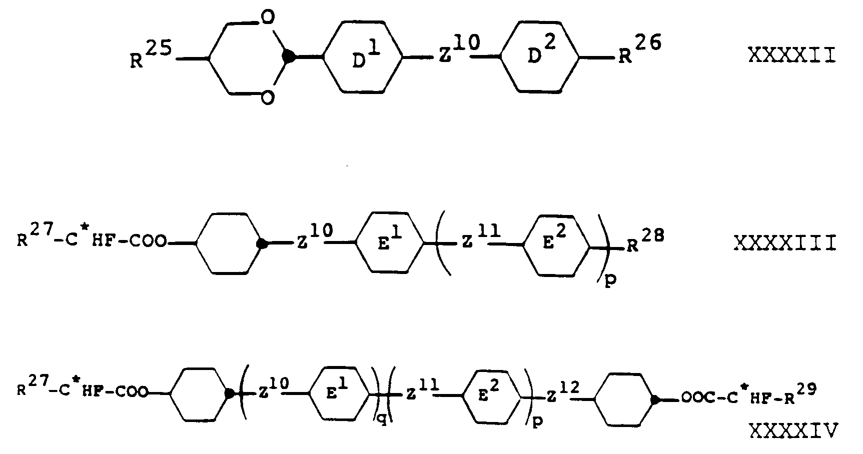
worin R¹⁹ und R²⁰ Alkyl, Alkoxy, Alkenyloxy, Alkanoyloxy oder Alkoxycarbonyl mit bis zu 18 Kohlenstoffatomen bezeichnen; r und s unabhängig voneinander 1 oder 2 bedeuten; R²¹ und R²² Alkyl, Alkoxy oder Alkenyloxy mit bis zu 18 Kohlenstoffatomen bezeichnen; Ring B unsubstituiertes oder mit Halogen und/ oder Methyl substituiertes 1,4-Phenylen darstellt; Ring C trans-1,4-Cyclohexylen oder unsubstituiertes oder mit Halogen und/oder Methyl substituiertes 1,4-Phenylen darstellt; p und q unabhängig voneinan- der für die Zahl O oder 1 stehen; R²³ und R²⁴ unabhängig voneinander eine unsubstituierte oder mit Halogen substituierte C₁-C₁₈-Alkyl- oder C₂-C₁₈-Alkenylgruppe bezeichnen, in welcher gegebenenfalls eine oder zwei nicht benachbarte CH₂-Gruppen durch -O-, -COO- und/oder -OOC- ersetzt sind; die Ringe D¹, D² und D³ unabhängig voneinander unsubstituiertes oder mit Cyano, Halogen oder Niederalkyl substituiertes 1,4-Phenylen darstellen; Z⁹ eine einfache Kovalenzbindung, -CH₂CH₂-, -OCH₂-, -COO- oder -OOC-bezeichnet; R²⁵ und R²⁶ unabhängig voneinander eine unsubstituierte oder halogensubstituierte C₁-C₁₈-Alkyl-oder C₂-C₁₈-Alkenylgruppe bedeuten, in welcher gegebenenfalls eine oder zwei nicht benachbarte CH₂-Gruppen durch Sauerstoff ersetzt sind; Ring D⁴ trans-1,4-Cyclohexylen oder unsubstituiertes oder mit Cyano, Halogen oder Niederalkyl substituiertes 1,4-Phenylen darstellt; Z¹⁰, Z¹¹ und Z¹² unabhängig voneinander eine einfache Kovalenzbindung, -COO-, -OOC-, -CH₂CH₂-, -OCH₂- oder -CH₂O- bezeichnen; R²⁷ und R²⁹ unabhängig voneinander eine C₁-C₁₅-Alkyl- oder C₂-C₁₅-Alkenylgruppe bedeuten, in welcher gegebenenfalls eine CH₂-Gruppe durch Sauerstoff ersetzt ist; R²⁸ eine unsubstituierte oder mit Halogen substituierte C₁-C₁₅-Alkyl- oder C₂-C₁₅-Alkylengruppe bezeichnet, in welcher gegebenenfalls eine CH₂-Gruppe durch Sauerstoff ersetzt ist und/oder gegebenenfalls eine CH₂-Gruppe durch eine Estergruppe -COO- oder -OOC- ersetzt ist; die Ringe E¹ und E² unabhängig voneinander unsubstituiertes oder mit Halogen, Cyano und/oder Methyl substituiertes 1,4-Phenylen darstellen, in welchem gegebenenfalls 1 oder 2 CH-Gruppen durch Stickstoff ersetzt sind; und C^{*} ein chirales Kohlenstoffatom bezeichnet.

Die Herstellung der flüssigkristallinen Gemische und der elektro-optischen Vorrichtungen kann in an sich bekannter Weise erfolgen.

Die Erfindung wird durch die folgenden Beispiele weiter veranschaulicht. Die optischen Antipoden von chiralen Verbindungen besitzen jeweils die gleichen Phasenumwandlungstemperaturen und absolut gleiche Werte der Verdrillung aber mit entgegengesetzten Vorzeichen. Die zur Charakterisierung der Phasenumwandlungen verwendeten Abkürzungen stehen für folgende Bedeutungen:
- C: für kristallin
- S: für smektisch
- S_{A}, S_{B}, S_{C} etc.: für smektisch A, B, C etc.
- S_{C}^{*}, S_{F}^{*} etc.: für chiral smektisch C, F etc.
- N: für nematisch
- N^{*}: für cholesterisch
- I: für isotrop.

### Beispiel 1

0,12 g Magnesiumspäne wurden vorgelegt und unter Stickstoffbegasung mit 5 ml absolutem Diäthyläther überschichtet und dann nach Zugabe eines Jodkristalls mit einer Lösung von 1,3 g 4-(Trifluormethoxy)benzylbromid in 25 ml Diäthyläther versetzt. Nach beendeter Zugabe wurde das Gemisch noch währen 30 Minuten zum Rückfluss erhitzt. Das Reaktionsgemisch wurde in einen Tropftrichter transferiert und dann bei 0°C und unter Stickstoffbegasung zu einer Lösung von 0,9 g Pivalinsäure-3E-[trans-4-(trans-4-[1E-Propenyl]cyclohexyl)cyclohexyl]-allylester, 0,03 g Kupfer-(I)-chlorid und 25 ml absolutem Diäthyläther zugetropft. Nach beendeter Zugabe wurde das Reaktionsgemisch noch während 30 Minuten bei Raumtemperatur gerührt, dann mit 20 ml 25%-iger Salzsäure versetzt und die organische Phase abgetrennt. Die wässrige Phase wurde dreimal mit je 50 ml Diäthyläther extrahiert. Die vereinigten organischen Phasen wurden nacheinander mit 500 ml Wasser, 250 ml konzentrierter Kaliumcarbonat-Lösung und nochmals mit 500 ml Wasser gewaschen, über Magnesiumsulfat getrocknet, filtriert und anschliessend eingeengt. Der Rückstand wurde an Kieselgel mit Hexan chromatographisch gereinigt. Umkristallisation aus Aceton ergab 0,5 g reines 1-Trifluormethoxy-4-[4E-(trans-4-(trans-4-[1E-Propenyl]-cyclohexyl)cyclohexyl)-3-butenyl]benzol mit Smp. (C-N) 63°C und Klp. (N-I) 149°C.

Der als Ausgangsmaterial verwendete Pivalinsäure-3E-[trans-4-(trans-4-[1E-Propenyl]cyclohexyl)cyclohexyl]-allylester wurde wie folgt hergestellt:
(a). Eine Lösung von 25 g trans-4-(trans-4-[1E-Propenyl]cyclohexyl)cyclohexancarbonitril in 50 ml Toluol wurde unter Stickstoffbegasung bei -78°C tropfenweise mit 150 ml einer 20%-igen Lösung (Gew./Vol.) von Diisobutylaluminiumhydrid in Toluol versetzt. Das Reaktionsgemisch wurde über Nacht bei Raumtemperatur gerührt, dann mit 500 ml Wasser versetzt und anschliessend viermal mit je 200 ml Dichlormethan extrahiert. Die vereinigten organischen Phasen wurden zweimal mit 500 ml Wasser gewaschen, über Magnesiumsulfat getrocknet, filtriert und anschliessend eingeengt. Dies ergab 20 g trans-4-(trans-4-[1E-Propenyl]-cyclohexyl)cyclohexancarboxaldehyd.
(b). Ein Gemisch von 10 g trans-4-(trans-4-[1E-Propenyl]cyclohexyl)cyclohexancarboxaldehyd, 12,5 g Aethyl-diäthylphosphonoacetat (C₂H₅O)₂PO-CH₂COOC₂H₅, 4,8 g Kaliumhydroxid und 100 ml Tetrahydrofuran wurde während 3 Stunden gerührt und dann mit 500 ml Wasser versetzt und anschliessend dreimal mit je 100 ml Diäthyläther extrahiert. Die vereinigten organischen Phasen wurden zweimal mit je 500 ml Wasser gewaschen, über Magnesiumsulfat getrocknet, filtriert und anschliessend eingeengt. Der Rückstand wurde an Kieselgel mit Toluol chromatographisch gereinigt. Umkristallisation aus Hexan ergab 12 g reinen 3E-[trans-4-(trans-4-[1E-Propenyl]cyclohexyl)-cyclohexyl]acrylsäureäthylester mit Smp. (C-N) 40°C und Klp. (N-I) 94°C.
(c). Eine Lösung von 5 g 3E-[trans-4-(trans-4-[1E-Propenyl]cyclohexyl)cyclohexyl]acrylsäure-äthylester in 100 ml Toluol wurde bei 0°C und unter Stickstoffbegasung tropfenweise mit 50 ml einer 20%-igen Lösung von Diisobutylaluminiumhydrid in Toluol versetzt. Nach beendeter Zugabe wurde die leicht gelbe Lösung noch 5 Stunden gerührt und dann vorsichtig mit 50 ml 25%-iger Salzsäure versetzt. Das Reaktionsgemisch wurde auf 100 ml Wasser gegossen und die organische Phase abgetrennt. Die wässrige Phase wurde zweimal mit je 100 ml Toluol nachextrahiert. Die vereinigten organischen Phasen wurden mit 500 ml Wasser, 500 ml konzentrierter Kaliumhydrogencarbonat-Lösung und nochmals mit 500 ml Wasser gewaschen und anschliessend über Magnesiumsulfat getrocknet, filtriert und eingeengt. Umkristallisation des Rückstandes aus Hexan ergab 4,2 g 3E-[trans-4-(trans-4-[1E-Propenyl]cyclohexyl)-cyclohexyl]allylalkohol mit Smp. (C-S_{B}) 56°C, S_{B}-N 95°C und Klp. (N-I) 113°C.
(d). 0,4 g Pivalinsäure, 1,0 g 3E-[trans-4-(trans-4-[1E-Propenyl]cyclohexyl)cyclohexyl]allylalkohol und 0,1 g 4-(Dimethylamino)pyridin wurden in 50 ml Dichlormethan gelöst und die Lösung innert 10 Minuten unter Rühren portionenweise mit 0,9 g N,N'-Dicyclohexylcarbodiimid versetzt. Das Gemisch wurde über Nacht bei Raumtemperatur gerührt und dann filtriert. Das Filtrat wurde mit Dichlormethan verdünnt, zweimal mit je 50 ml gesättigter Natriumcarbonat-Lösung und dann mit Wasser gewaschen, über Magnesiumsulfat getrocknet, filtriert und dann eingeengt. Chromatographie des Rückstandes an Kieselgel mit Toluol ergab 1,3 g Pivalinsäure-3E-[trans-4-(trans-4-[1E-Propenyl]cyclohexyl)cyclohexyl]allylester.

In analoger Weise können folgende Verbindungen hergestellt werden:
1-Fluor-4-[4E-(trans-4-pentylcyclohexyl)-3-butenyl]-benzol, Smp. (C-I) -6°C, Klp. (N-I) -13°C;
1-Chlor-4-[4E-(trans-4-pentylcyclohexyl)-3-butenyl]-benzol;
1-Brom-4-[4E-(trans-4-pentylcyclohexyl)-3-butenyl]-benzol;
1-Jod-4-[4E-(trans-4-pentylcyclohexyl)-3-butenyl]-benzol;
1,2-Difluor-4-[4E-(trans-4-pentylcyclohexyl)-3-butenyl]benzol, Smp. (C-I) -11°C, Klp. (C-I) -33°C;
1-Methyl-4-[4E-(trans-4-pentylcyclohexyl)-3-butenyl]-benzol;
1-(Trifluormethoxy)-4-[4E-(trans-4-pentylcyclohexyl)-3-butenyl]benzol, Smp. (C-I) 5°C, Klp. (N-I) -17°C;
1-Fluor-4-[4E-(trans-4-[trans-4-propylcyclohexyl]-cyclohexyl)-3-butenyl]benzol, Smp. (C-S_{B}) 33°C, S_{B}-N 74°C, Klp. (N-I) 135°C;
1,2-Difluor-4-[4E-(trans-4-[trans-4-propylcyclohexyl]cyclohexyl)-3-butenyl]benzol, Smp. (C-S_{B}) 27°C, S_{B}-N 50°C, Klp. (N-I) 119°C;
1-Methyl-4-[4E-(trans-4-[trans-4-propylcyclohexyl]-cyclohexyl)-3-butenyl]benzol;
1-(Trifluormethyl)-4-[4E-(trans-4-[trans-4-propylcyclohexyl]cyclohexyl)-3-butenyl]benzol;
1-(Trifluormethoxy)-4-[4E-(trans-4-[trans-4-propylcyclohexyl]cyclohexyl)-3-butenyl]benzol, Smp. (C-S_{B}) 41°C, S_{B}-N 83°C, Klp. (N-I) 128°C;
1-Fluor-4-[4E-(trans-4-]trans-4-(1E-propenyl)cyclohexyl]cyclohexyl)-3-butenyl]benzol, Smp. (C-N) 52°C, Klp. (N-I) 159°C;
1,2-Difluor-4-[4E-(trans-4-[trans-4-(1E-propenyl)-cyclohexyl]cyclohexyl)-3-butenyl]benzol, Smp. (C-N) 60°C, Klp. (N-I) 140°C;
1-Aethoxy-2,3-difluor-4-[4E-(trans-4-[trans-4-propylcyclohexyl]cyclohexyl)-3-butenyl]benzol;
1-Fluor-4-[4E-(trans-4-]trans-4-pentylcyclohexyl]-cyclohexyl)-3-butenyl]benzol;
1,2-Difluor-4-[4E-(trans-4-]trans-4-pentylcyclohexyl]cyclohexyl)-3-butenyl]benzol;
1-Methyl-4-[4E-(trans-4-]trans-4-pentylcyclohexyl]-cyclohexyl)-3-butenyl]benzol;
1-(Trifluormethyl)-4-[4E-(trans-4-[trans-4-pentylcyclohexyl]cyclohexyl)-3-butenyl]benzol;
1-(Trifluormethoxy)-4-[4E-(trans-4-[trans-4-pentylcyclohexyl]cyclohexyl)-3-butenyl]benzol;
1-Aethoxy-2,3-difluor-4-[4E-(trans-4-[trans-4-pentylcyclohexyl]cyclohexyl)-3-butenyl]benzol;
1-(trans-4-Propylcyclohexyl)-4-[4E-(trans-4-propylcyclohexyl)-3-butenyl]benzol;
1-(trans-4-Propylcyclohexyl)-4-[4E-(trans-4-pentylcyclohexyl)-3-butenyl]benzol;
1-(trans-4-Pentylcyclohexyl)-4-[4E-(trans-4-propylcyclohexyl)-3-butenyl]benzol;
4-(trans-4-Propylcyclohexyl)-4'-[4E-(trans-4-propylcyclohexyl)-3-butenyl]biphenyl;
4-(trans-4-Propylcyclohexyl)-4'-[4E-(trans-4-pentylcyclohexyl)-3-butenyl]biphenyl;
4-[2(S)-Methylbutyloxy]-4'-[4E-(trans-4-pentylcyclohexyl)-3-butenyl]biphenyl;
1-[2-(trans-4-Pentylcyclohexyl)-1-äthyl]-4-[4E-(trans-4-pentylcyclohexyl)-3-butenyl]benzol;
2-(4-[4E-(trans-4-Pentylcyclohexyl)-3-butenyl]phenyl)-5-propylpyridin;
2-(4-[4E-(trans-4-Pentylcyclohexyl)-3-butenyl]phenyl)-5-butylpyridin;
2-(4-[4E-(trans-4-Pentylcyclohexyl)-3-butenyl]phenyl)-5-pentylpyridin;
2-(4-[4E-(trans-4-Pentylcyclohexyl)-3-butenyl]phenyl)-5-hexylpyridin;
2-(4-[4E-(trans-4-Pentylcyclohexyl)-3-butenyl]phenyl)-5-heptylpyridin;
2-(4-[4E-(trans-4-Pentylcyclohexyl)-3-butenyl]phenyl)-5-octylpyridin;
2-(4-[4E-(trans-4-Pentylcyclohexyl)-3-butenyl]phenyl)-5-nonylpyridin;
2-(4-[4E-(trans-4-Pentylcyclohexyl)-3-butenyl]phenyl)-5-decylpyridin;
5-Pentyl-2-[4-[4E-(trans-4-Pentylcyclohexyl)-3-butenyl]phenyl]pyrimidin;
5-Hexyl-2-[4-[4E-(trans-4-Pentylcyclohexyl)-3-butenyl]phenyl]pyrimidin;
5-Heptyl-2-[4-[4E-(trans-4-Pentylcyclohexyl)-3-butenyl]phenyl]pyrimidin;
5-Octyl-2-[4-[4E-(trans-4-Pentylcyclohexyl)-3-butenyl]phenyl]pyrimidin;
5-Nonyl-2-[4-[4E-(trans-4-Pentylcyclohexyl)-3-butenyl]phenyl]pyrimidin;
5-Decyl-2-[4-[4E-(trans-4-Pentylcyclohexyl)-3-butenyl]phenyl]pyrimidin;
5-Undecyl-2-[4-[4E-(trans-4-Pentylcyclohexyl)-3-butenyl]phenyl]pyrimidin;
1-(4-[4E-(trans-4-Pentylcyclohexyl)-3-butenyl]phenyl)-4-pentylbicyclo[2.2.2]octan;
(4aαH,8aβH)-Decahydro-2α-(4-[4E-(trans-4-Pentylcyclohexyl)-3-butenyl]phenyl)-6β-pentylnaphthalin.

### Beispiel 2

Ein Gemisch von 0,3 g 4-[4E-(trans-4-Pentylhexyl)-3-butenyl]phenol, 0,14 g Methyljodid, 0,6 g Kaliumcarbonat und 50 ml absolutem Butanon wurde über Nacht zum Rückfluss erhitzt. Anschliessend wurde das abgekühlte Reaktionsgemisch auf Wasser gegossen und dreimal mit je 50 ml Diäthyläther extrahiert. Die vereinigten organischen Phasen wurden mit 500 ml Wasser gewaschen, über Magnesiumsulfat getrocknet, filtriert und eingeengt. Chromatographie des Rückstandes an Kiesel mit Toluol und Umkristallisation aus Aethanol ergab reines 1-Methoxy-4-[4E-(trans-4-pentylcyclohexyl)-3-butenyl]benzol mit Smp. (C-N) 25°C und Klp. (N-I) 34°C.

Das als Ausgangsmaterial verwendete 4-[4E-(trans-4-Pentylcyclohexyl)-3-butenyl]phenol wurde wie folgt hergestellt:
(a). Ein Gemisch von 10 g 3-(4-Hydroxyphenyl)-1-propanol, 19 g Triphenylphosphin und 200 ml Dichlormethan wurde bei -18°C tropfenweise mit einer Lösung von 24 g Tetrabrommethan in 100 ml Dichlormethan versetzt. Das Reaktionsgemisch wurde über Nacht bei Raumtemperatur gerührt und dann eingeengt. Der Rückstand wurde an Kieselgel mit Toluol/Aethylacetat (Vol. 4:1) chromatographisch gereinigt. Dies ergab 14 g 3-(4-Hydroxyphenyl)-1-propylbromid.
(b). 5,0 g 3-(4-Hydroxyphenyl)-1-propylbromid, 2,3 g 2,3-Dihydro-2H-pyran, 0,1 g Bis-(trimethylsilyl)-sulfat und 100 ml absolutes Dichlormethan wurden vorgelegt und eine Stunde bei Raumtemperatur gerührt. Anschliessend wurde das Reaktionsgemisch mit 0,5 ml absolutem Pyridin versetzt und dann eingeengt. Der flüssige Rückstand wurde an Kieselgel mit Toluol chromatographisch gereinigt. Dies ergab 4,5 g 3-(4-[Tetrahydro-2-pyranyloxy]phenyl)-1-propylbromid.
(c). Ein Gemisch von 3,0 g 3-(4-[Tetrahydro-2-pyranyloxy]phenyl)-1-propylbromid, 3,2 g Triphenylphosphin und 5 ml absolutem N,N'-Dimethylformamid wurde über Nacht unter Stickstoffbegasung und leichtem Rückfluss erwärmt. Der gebildete Niederschlag wurde abfiltriert und das abgekühlte Filtrat mit 10 ml Aethylacetat versetzt, während 30 Minuten bei 0°C stehengelassen und dann nochmals filtriert. Die vereinigten Feststoffe wurden unter Vakuum getrocknet. Dies ergab 4,4 g [3-(4-[Tetrahydro-2-pyranyloxy]phenyl)-1-propyl]triphenylphosphoniumbromid mit Smp. 224 - 226°C.
(d). Ein Gemisch von 4,0 g [3-(4-[Tetrahydro-2-pyranyloxy]phenyl)-1-propyl]triphenylphosphoniumbromid, 1,3 g trans-4-Pentylcyclohexancarboxaldehyd und 50 ml absolutem Tetrahydrofuran wurde portionenweise mit 0,8g festem Kalium-t-butylat versetzt. Nach beendeter Zugabe wurde das Reaktionsgemisch noch zwei Stunden gerührt und dann auf 500 ml Wasser gegossen und dreimal mit je 100 ml Diäthyläther extrahiert. Die organischen Phasen wurden noch zweimal mit je 500 ml konzentrierter Kochsalz-Lösung gewaschen, über Magnesiumsulfat getrocknet, filtriert und anschliessend eingeengt. Chromatographie des Rückstandes an Kieselgel mit Toluol ergab 1,0 g 1-[Tetrahydro-2-pyranyloxy]-4-[4-(trans-4-pentylcyclohexyl)-3-butenyl]-benzol (cis/trans-Gemisch).
e). Eine Lösung von 1,0 g 1-[Tetrahydro-2-pyranyloxy]-4-[4-(trans-4-pentylcyclohexyl)-3-butenyl]benzol, 0,02 g Bis-(trimethylsilyl)-sulfat und 25 ml Methanol wurde während zwei Stunden gerührt. Anschliessend wurde das Reaktionsgemisch mit 0,1 ml Pyridin versetzt und dann eingeengt. Der flüssige Rückstand wurde an Kieselgel mit Toluol chromatographisch gereinigt. Dies ergab 0,9 g 4-[4-(trans-4-Pentylcyclohexyl)-3-butenyl]phenol (cis/trans-Gemisch).
(f). Eine Lösung von 0,9 g 4-[4-(trans-4-Pentylcyclohexyl)-3-butenyl]phenol in 50 ml Aethanol wurde mit einer Lösung von 0,2 g Benzolsulfinsäure-Natriumsalz in 1 ml Wasser und zwei Tropfen konzentrierter Salzsäure versetzt. Das Reaktionsgemisch wurde über Nacht auf 65°C erwärmt, dann mit 100 ml Wasser versetzt und dreimal mit je 50 ml Diäthyläther extrahiert. Die vereinigten organischen Phasen wurden zweimal mit je 250 ml konzentrierter Kochsalz-Lösung gewaschen, über Magnesiumsulfat getrocknet, filtriert und anschliessend eingeengt. Umkristallisation des Rückstandes aus Hexan ergab 0,7 g 4-[4E-(trans-4-Pentylcyclohexyl)-3-butenyl]phenol mit Smp. 89 - 90°C.

In analoger Weise können folgende Verbindungen hergestellt werden;
1-Aethoxy-4-[4E-(trans-4-propylcyclohexyl)-3-butenyl]benzol;
1-Propyloxy-4-[4E-(trans-4-propylcyclohexyl)-3-butenyl]benzol;
1-Pentyloxy-4-[4E-(trans-4-propylcyclohexyl)-3-butenyl]benzol;
1-Hexyloxy-4-[4E-(trans-4-propylcyclohexyl)-3-butenyl]benzol; Smp. (C-N) 28°C, S_{B}-N 36°C, Klp. (N-I) 40°C;
1-Heptyloxy-4-[4E-(trans-4-propylcyclohexyl)-3-butenyl]benzol;
1-Octyloxy-4-[4E-(trans-4-propylcyclohexyl)-3-butenyl]benzol;
1-Aethoxy-4-[4E-(trans-4-pentylcyclohexyl)-3-butenyl]benzol, Smp. (C-N) 34°C, Klp. (N-I) 51°C;
1-Propyloxy-4-[4E-(trans-4-pentylcyclohexyl)-3-butenyl]benzol, Smp. (C-S) 32°C, Klp. (S_{B}-I) 42°C;
1-Butyloxy-4-[4E-(trans-4-pentylcyclohexyl)-3-butenyl]benzol, Smp. (C-S_{B}) 24°C, Klp. (S_{B}-I) 58°;
1-Pentyloxy-4-[4E-(trans-4-pentylcyclohexyl)-3-butenyl]benzol, Smp. (C-S_{B}) 28°C, Klp. (S_{B}-I) 52°C;
1-Hexyloxy-4-[4E-(trans-4-pentylcyclohexyl)-3-butenyl]benzol, Smp. (C-S_{B}) 32°C, Klp. (S_{B}-I) 56°C;
1-Heptyloxy-4-[4E-(trans-4-pentylcyclohexyl)-3-butenyl]benzol;
1-Octyloxy-4-[4E-(trans-4-pentylcyclohexyl)-3-butenyl]benzol;
1-[(trans-4-Pentylcyclohexyl)methoxy]-4-[4E-(trans-4-pentylcyclohexyl)-3-butenyl]benzol, Smp. (C-S_{B}) 48°C, Klp. (S_{B}-I) 125°C;
1-[3-(trans-4-Pentylcyclohexyl)-1-propyloxy]-4-[4E-(trans-4-pentylcyclohexyl)-3-butenyl]benzol, Smp. (C-S_{B}) 110°C, S _{B}-N 114°C, Klp. (N-I) 122°C;
1-[3E-(trans-4-Pentylcyclohexyl)allyloxy]-4-[4E-(trans-4-pentylcyclohexyl)-3-butenyl]benzol, Smp. (C-S_{Z}) 78°C, S_{Z}-S_{B} 100°C, S_{B}-N 119°C, Klp. (N-I) 116°C;

### Beispiel 3

1,7 g 2,3-Difluor-4-dodecyloxybenzoesäure, 1,5 g 4-[4E-(trans-4-Pentylcyclohexyl)-3-butenyl]phenol und 0,1 g 4-(Dimethylamino)pyridin wurden in 250 ml Dichlormethan gelöst und die Lösung innert 10 Minuten unter Rühren portionenweise mit 1,2 g N,N'-Dicyclohexylcarbodiimid versetzt. Das Gemisch wurde über Nacht bei Raumtemperatur gerührt und dann filtriert. Das Filtrat wurde mit Dichlormethan verdünnt, zweimal mit je 50 ml gesättigter Natriumcarbonat-Lösung und dann mit 100 ml Wasser gewaschen, über Magnesiumsulfat getrocknet, filtriert und dann eingeengt. Chromatographie des Rückstandes an Kieselgel mit Toluol und Umkristallisation aus Aethanol ergab 2 g 2,3-Difluor-4-dodecyloxybenzoesäure-4-[4E-(trans-4-pentylcyclohexyl)-3-butenyl]phenylester mit Smp. (C-S_{C}) 51°C, S_{C}-N 115°C und Klp. (N-I) 133°C.

In analoger Weise können folgende Verbindungen hergestellt werden:
2,3-Difluor-4-heptyloxybenzoesäure-4-[4E-(trans-4-pentylcyclohexyl)-3-butenyl]phenylester, Smp. (C-S_{C}) 55°C, S_{C}-N 77°C, Klp. (N-I) 140°C;
2,3-Difluor-4-octyloxybenzoesäure-4-[4E-(trans-4-pentylcyclohexyl)-3-butenyl]phenylester, Smp. (C-S_{C}) 75°C, S_{C}-N 88°C, Klp. (N-I) 140°C;
2,3-Difluor-4-nonyloxybenzoesäure-4-[4E-(trans-4-pentylcyclohexyl)-3-butenyl]phenylester, Smp. (C-S_{C}) 77°C, S_{C}-N 99°C, Klp. (N-I) 137°C;
2,3-Difluor-4-decyloxybenzoesäure-4-[4E-(trans-4-pentylcyclohexyl)-3-butenyl]phenylester, Smp. (C-S_{C}) 72°C, S_{C}-N 106°C, Klp. (N-I) 136°C;
2,3-Difluor-4-undecyloxybenzoesäure-4-[4E-(trans-4-pentylcyclohexyl)-3-butenyl]phenylester, Smp. (C-S_{C}) 60°C, S_{C}-N 111°C, Klp. (N-I) 134°C;
trans-4-Propylcyclohexancarbonsäure-4-[4E-(trans-4-pentylcyclohexyl)-3-butenyl]phenylester;
trans-4-Pentylcyclohexancarbonsäure-4-[4E-(trans-4-pentylcyclohexyl)-3-butenyl]phenylester;
trans-4-Heptylcyclohexancarbonsäure-4-[4E-(trans-4-pentylcyclohexyl)-3-butenyl]phenylester.

### Beispiel 4

Ein Gemisch von 14 g 3-(4-Cyanophenyl)propionaldehyd, 54 g (trans-4-Pentylcyclohexyl)methyl-triphenylphosphoniumbromid, 11 g Kalium-t-butylat und 500 ml absolutem Tetrahydrofuran wurde in analoger Weise zu Beispiel 2 (d) umgesetzt. Hierbei wurden 16 g 4-[4-(trans-4-Pentylcyclohexyl)-3-butenyl]benzonitril als cis/trans-Gemisch erhalten.

Ein Gemisch von 16 g 4-[4-(trans-4-Pentylcyclohexyl)-3-butenyl]benzonitril (cis/trans-Gemisch), 50 ml Aethanol und 1,9 g Benzolsulfinsäure (frisch hergestellt aus 13 ml Wasser, 2,0 g Benzolsulfinsäure-Natriumsalz und 5 Tropfen konzentrierter Salzsäure) wurde in analoger Weise zu Beispiel 2 (f) umgesetzt. Hierbei wurden 11 g 4-[4E-(trans-4-Pentylcyclohexyl)-3-butenyl]benzonitril erhalten; Smp. (C-N) 40°C, Klp. (N-I) 45°C.

Der als Ausgangsmaterial verwendete 3-(4-Cyanophenyl)propionaldehyd wurde wie folgt hergestellt:
(a). Ein Gemisch von 20 g 4-Cyanobenzaldehyd, 79 g (1,3-Dioxolan-2-yl)methyl-triphenylphosphoniumbromid, 19 g Kalium-t-butylat und 500 ml absolutem Tetrahydrofuran wurden in analoger Weise zu Beispiel 2 (d) umgesetzt. Hierbei wurden 20 g 4-[2-(1,3-Dioxolan-2-yl)vinyl]benzonitril als cis/trans-Gemisch erhalten.
(b). Ein Gemisch von 20 g 4-[2-(1,3-Dioxolan-2-yl)-vinyl]benzonitril (cis/trans-Gemisch), 200 ml absolutem Toluol und 100 ml absolutem Aethylacetat wurde mit 2 g Palladium/Kohle (10%) versetzt und bei Normaldruck und Raumtemperatur bis zum Stillstand hydriert. Das anorganische Material wurde abfiltriert und das Filtrat eingeengt. Der Rückstand wurde an Kieselgel mit Toluol/Aethylacetat (Vol. 4:1) chromatographisch gereinigt. Dies ergab 19 g 4-[2-(1,3-Dioxolan-2-yl)äthyl]benzonitril.
(c). Ein Gemisch von 19 g 4-[2-(1,3-Dioxolan-2-yl)-äthyl]benzonitril, 10 ml Ameisensäure und 100 ml absolutem Toluol wurde über Nacht bei Raumtemperatur gerührt. Das Reaktionsgemisch wurde mit 200 ml Wasser und dann mit 75 ml gesättigter Natriumcarbonat-Lösung gewaschen, über Magnesiumsulfat getrocknet, filtriert und anschliessend eingeengt. Dies ergab 14 g 3-(4-Cyanophenyl)propionaldehyd.

In analoger Weise können folgende Verbindungen hergestellt werden:
4-[4E-(trans-4-Methylcyclohexyl)-3-butenyl]benzonitril;
4-[4E-(trans-4-Aethylcyclohexyl)-3-butenyl]benzonitril;
4-[4E-(trans-4-Propylcyclohexyl)-3-butenyl]benzonitril;
4-[4E-(trans-4-Butylcyclohexyl)-3-butenyl]benzonitril;
4-[4E-(trans-4-Hexylcyclohexyl)-3-butenyl]benzonitril;
4-[4E-(trans-4-Heptylcyclohexyl)-3-butenyl]benzonitril;
4-[4E-(trans-4-Octylcyclohexyl)-3-butenyl]benzonitril;
4-[4E-(trans-4-Propylcyclohexyl)-3-butenyl]-4'-cyanobiphenyl;
4-[4E-(trans-4-Pentylcyclohexyl)-3-butenyl]-4'-cyanobiphenyl;
4-[4E-(trans-4-Heptylcyclohexyl)-3-butenyl]-4'-cyanobiphenyl.

### Beispiel 5

Eine Lösung von 3 g 4-[4E-(trans-4-Pentylcyclohexyl)-3-butenyl]benzaldehyd und 1,7 g 2-Pentyl-1,3-propandiol in 50 ml Toluol wurde mit 2 Tropfen 10%iger (Vol.) Schwefelsäure versetzt. Das Gemisch wurde 2 Stunden zum Sieden erhitzt, wobei das entstandene Wasser gleichzeitig abdestilliert wurde. Dann wurden 4 Tropfen Triäthylamin zum Reaktionsgemisch zugegeben. Nach dem Erkalten wurde das Gemisch mit 50 ml 1N Natriumhydrogencarbonat-Lösung und zweimal mit je 50 ml Wasser gewaschen, über Magnesiumsulfat getrocknet, filtriert und anschliessend eingeengt. Chromatographie des Rückstandes an Kieselgel mit Toluol und Umkristallisation aus Aceton ergab 1,2 g trans-5-Pentyl-2-(4-[4E-(trans-4-pentylcyclohexyl)-3-butenyl]-phenyl-1,3-dioxan.

Der als Ausgangsmaterial verwendete 4-[4E-(trans-4-Pentylcyclohexyl)-3-butenyl]benzaldehyd wurde wie folgt hergestellt:
Eine Lösung von 10 g 4-[4E-(trans-4-Pentylcyclohexyl)-3-butenyl]benzonitril in 200 ml absolutem Dichlormethan wurde bei -78°C und unter Stickstoffbegasung mit 50 ml einer Lösung von Diisobutylaluminiumhydrid in Hexan (Vol. 20%) versetzt. Nach beendeter Zugabe wurde das Reaktionsgemisch noch 8 Stunden bei Raumtemperatur gerührt und dann mit 200 ml Wasser versetzt und dreimal mit je 100 ml Dichlormethan extrahiert. Die vereinigten organischen Phasen wurden zweimal mit je 500 ml Wasser gewaschen, über Magnesiumsulfat getrocknet, filtriert und eingeengt. Dies ergab 7,5 g 4-[4E-(trans-4-Pentylcyclohexyl)-3-butenyl]-benzaldehyd.

In analoger Weise können folgende Verbindungen hergestellt werden:
trans-5-Propyl-2-(4-[4E-(trans-4-propylcyclohexyl)-3-butenyl]phenyl)-1,3-dioxan;
trans-5-Propyl-2-(4-[4E-(trans-4-pentylcyclohexyl)-3-butenyl]phenyl)-1,3-dioxan;
trans-5-Pentyl-2-(4-[4E-(trans-4-propylcyclohexyl)-3-butenyl]phenyl)-1,3-dioxan.

### Beispiel 6

2 g 4-[4E-(trans-4-Pentylcyclohexyl)-3-butenyl]benzoesäure, 0,7 g 4-Fluorphenol, 1,5 g N,N'-Dicyclohexylcarbodiimid, 0,1 g 4-(Dimethylamino)pyridin und 100 ml Dichlormethan wurden in analoger Weise zu Beispiel 3 umgesetzt. Dies ergab 1,8 g 4-[4E-(trans-4-Pentylcyclohexyl)-3-butenyl]-benzoesäure-4-fluorphenylester, Smp. (C-N) 65°C, Klp. (N-I) 123°C.

Die als Ausgangsmaterial verwendete 4-[4E-(trans-4-Pentylcyclohexyl)-3-butenyl]benzoesäure wurde wie folgt hergestellt:
Eine Lösung von 4 g 4-[4E-(trans-4-Pentylcyclohexyl)-3-butenyl]benzaldehyd (hergestellt nach Beispiel 5) in 100 ml Aceton wurde tropfenweise mit 10 ml Jones' Reagenz versetzt. Das Gemisch wurde 1 Stunde bei Raumtemperatur gerührt und dann auf 100 ml Wasser gegossen. Der dabei entstandene Niederschlag wurde abfiltriert, portionenweise mit Wasser gewaschen und am Vakuum getrocknet. Das Rohprodukt wurde aus Aethanol umkristallisiert und ergab 2,5 g reine 4-[4E-(trans-4-Pentylcyclohexyl)-3-butenyl]benzoesäure.

In analoger Weise können folgende Verbindungen hergestellt werden:
4-[4E-(trans-4-Pentylcyclohexyl)-3-butenyl]benzoesäure-4-chlorphenylester;
4-[4E-(trans-4-Pentylcyclohexyl)-3-butenyl]benzoesäure-4-bromphenylester;
4-[4E-(trans-4-Pentylcyclohexyl)-3-butenyl]benzoesäure-4-jodphenylester;
4-[4E-(trans-4-Pentylcyclohexyl)-3-butenyl]benzoesäure-4-cyanophenylester, Smp. (C-N) 76°C, Klp. (N-I) 171°C;
4-[4E-(trans-4-Pentylcyclohexyl)-3-butenyl]benzoesäure-2-fluor-4-cyanophenylester;
4-[4E-(trans-4-Pentylcyclohexyl)-3-butenyl]benzoesäure-3-fluor-4-cyanophenylester, Smp. (C-N) 63°C, Klp. (N-I) 149°C;
4-[4E-(trans-4-Pentylcyclohexyl)-3-butenyl]benzoesäure-3,4-difluorphenylester, Smp. (C-N) 40°C, Klp. (N-I) 104°C;
4-[4E-(trans-4-Pentylcyclohexyl)-3-butenyl]benzoesäure-4-trifluormethoxyphenylester, Smp. (C-N) 70°C, Klp. (N-I) 128°C;
4-[4E-(trans-4-Pentylcyclohexyl)-3-butenyl]benzoesäure-2,3-difluor-4-äthoxyphenylester;
4-[4E-(trans-4-Pentylcyclohexyl)-3-butenyl]benzoesäure-2,3-dicyano-4-pentylphenylester;
4-[4E-(trans-4-Pentylcyclohexyl)-3-butenyl]benzoesäure-4-methylphenylester;
4-[4E-(trans-4-Pentylcyclohexyl)-3-butenyl]benzoesäure-4-propylphenylester;
4-[4E-(trans-4-Pentylcyclohexyl)-3-butenyl]benzoesäure-4-pentylphenylester;
4-[4E-(trans-4-Pentylcyclohexyl)-3-butenyl]benzoesäure-trans-4-propylcyclohexylester.

## Patentansprüche

1. Verbindungen der allgemeinen Formel 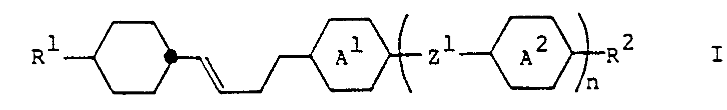 worin n für die Zahl O oder 1 steht; R¹ eine Gruppe R³ oder R³-A³-Z²- und R² eine Gruppe R⁴ oder R⁴-A⁴-Z³ - bezeichnen; Ring A¹ unsubstituiertes oder mit Halogen, Cyano und/oder Methyl substituiertes 1,4-Phenylen bedeutet, in welchem gegebenenfalls 1 oder 2 CH-Gruppen durch Stickstoff ersetzt sind; A³, A⁴ und Ring A² unabhängig voneinander unsubstituiertes oder mit Halogen, Cyano und/oder Methyl substituiertes 1,4-Phenylen, in welchem gegebenenfalls 1 oder 2 CH-Gruppen durch Stickstoff ersetzt sind, oder trans-1,4-Cyclohexylen, trans-1,3-Dioxan-2,5-diyl, 1-Cyano-trans-1,4-cyclohexylen, Bicyclo[2.2.2]octan-1,4-diyl, Naphthalin-2,6-diyl, Tetralin-2,6-diyl oder trans-Decalin-2,6-diyl darstellen; Z¹, Z² und Z³ unabhängig voneinander eine einfache Kovalenzbindung, -COO-, -OOC-, -CH₂O-, -OCH₂-, -CH₂CH₂-, -C≡C-, -(CH₂)₃O-, -O(CH₂)₃-, -(CH₂)₄- oder die trans-Form von -CH=CH-CH₂CH₂-, -CH₂CH₂CH=CH-, -CH=CH-CH₂O- oder -OCH₂-CH=CH- bedeuten; R³ und R⁴ unabhängig voneinander Halogen, Cyano, -NCS, -CF₃, -OCF₃ oder Alkyl bezeichnen, in welchem gegebenenfalls eine >CH-CH< durch >C=C< ersetzt ist und/oder gegebenenfalls eine oder zwei nicht benachbarte Methylengruppen durch -O-, -COO- und/oder -OOC- ersetzt sind und/oder gegebenenfalls eine Methylengruppe durch -CHX- ersetzt ist; und X Halogen, Cyano oder Methyl bedeutet,
mit Ausnahme von 1,2,6-Trifluor-4-[4E-(trans-4-pentylcyclohexyl)-3-butenyl]benzol.

2. Verbindungen nach Anspruch 1, gekennzeichnet durch die allgemeine Formel 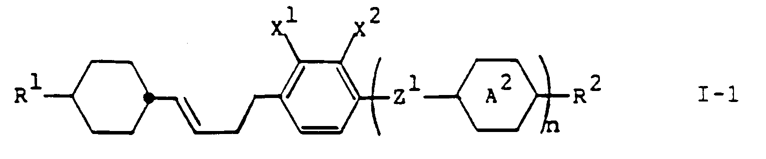 worin n, R¹, R², Z¹ und Ring A² die in Anspruch 1 gegebenen Bedeutungen haben und X¹ und X² unabhängig voneinander Wasserstoff, Halogen, Cyano oder Methyl bezeichnen.

3. Verbindungen nach Anspruch 1 oder 2, dadurch gekennzeichnet, dass A³ und A⁴ unabhängig voneinander trans-1,4-Cyclohexylen oder unsubstituiertes oder mit Halogen, Cyano und/oder Methyl substituiertes 1,4-Phenylen darstellen.

4. Verbindungen nach einem der Ansprüche 1 bis 3, dadurch gekennzeichnet, dass Ring A² für unsubstituiertes oder mit Halogen, Cyano und/oder Methyl monosubstituiertes oder 2,3-disubstituiertes 1,4-Phenylen, für trans-1,4-Cyclohexylen, trans-1,3-Dioxan-2,5-diyl, 1-Cyano-trans-1,4-cyclohexylen, Bicyclo[2.2.2]octan-1,4-diyl, Naphthalin-2,6-diyl, Tetralin-2,6-diyl, trans-Decalin-2,6-diyl, Pyridin-2,5-diyl, Pyrimidin-2,5-diyl oder Pyrazin-2,5-diyl steht.

5. Verbindungen nach einem der Ansprüche 1 bis 4, dadurch gekennzeichnet, dass Z¹, Z² und Z³ je eine einfache Kovalenzbindung bedeuten oder eine der Gruppen Z¹, Z² und Z³ auch -COO-, -OOC-, -CH₂O-, -OCH₂-, -CH₂CH₂-, -C≡C-, -(CH₂)₃O-, -O(CH₂)₃-, -(CH₂)₄- oder die trans-Form von -CH=CH-CH₂CH₂-, -CH₂CH₂-CH=CH-, -CH=CH-CH₂O- oder -OCH₂-CH=CH- bedeutet.

6. Verbindungen nach einem der Ansprüche 1 bis 5, gekennzeichnet durch die allgemeinen Formeln 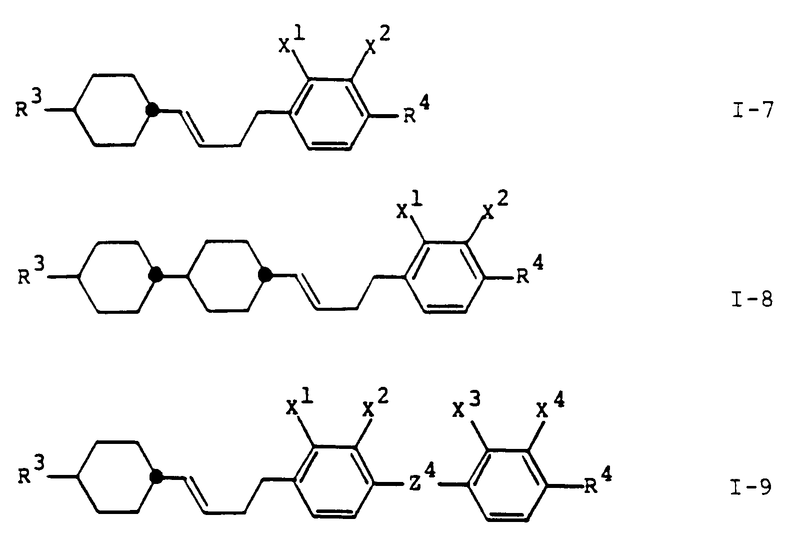 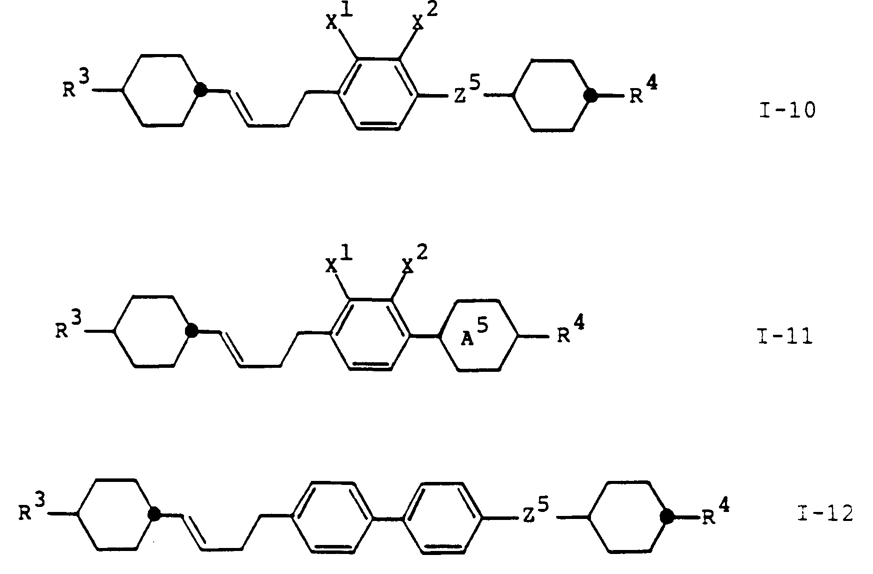 worin R³ und R⁴ die in Anspruch 1 gegebenen Bedeutungen haben; X¹, X², X³ und X⁴ unabhängig voneinander Wasserstoff, Halogen, Cyano oder Methyl bezeichnen; Z⁴ eine einfache Kovalenzbindung, -COO-, -OOC- oder -C≡C- bedeutet; Z⁵ eine einfache Kovalenzbindung, -OOC-, -OCH₂- -CH₂CH₂-, -C≡C-, -O(CH₂)₃-, -(CH₂)₄- oder die trans-Form von -CH₂CH₂-CH=CH- oder -OCH₂-CH=CH- bezeichnet; und Ring A⁵ Pyridin-2,5-diyl, Pyrimidin-2,5-diyl, Pyrazin-2,5-diyl, trans-1,3-Dioxan-2,5-diyl, Bicyclo[2.2.2]octan-1,4-diyl, Naphthalin-2,6-diyl, Tetralin-2,6-diyl oder trans-Decalin-2,6-diyl darstellt.

7. Verbindungen nach Anspruch 2 oder 6, dadurch gekennzeichnet, dass X¹, X², X³ und X⁴ unabhängig voneinander Wasserstoff oder Fluor bedeuten.

8. Verbindungen nach einem der Ansprüche 1 bis 7, dadurch gekennzeichnet, dass R³ und R⁴ unabhängig voneinander Alkyl bezeichnen, in welchem gegebenenfalls eine >CH-CH< durch >C=C< ersetzt ist und/oder gegebenenfalls eine oder zwei nicht benachbarte Methylengruppen durch -O-, -COO- und/oder -OOC- ersetzt sind und/oder gegebenenfalls eine Methylengruppe durch -CHX- ersetzt ist, oder R⁴ auch Halogen, Cyano, -NCS, -CF₃ oder -OCF₃ bezeichnet.

9. Verbindungen nach einem der Ansprüche 1 bis 8, dadurch gekennzeichnet, dass R³ und R⁴ höchstens je 18, vorzugsweise höchstens je 12 Kohlenstoffatome aufweisen.

10. Verbindungen nach einem der Ansprüche 1 bis 9, dadurch gekennzeichnet, dass R³ Alkyl, Alkenyl, Alkoxy, Alkenyloxy, Alkoxycarbonyl, Alkenyloxycarbonyl, Alkanoyloxy oder Alkenoyloxy, vorzugsweise Alkyl oder Alkenyl bedeutet.

11. Verbindungen nach einem der Ansprüche 1 bis 10, dadurch gekennzeichnet, dass R⁴ Alkyl, Alkenyl, Alkoxy, Alkenyloxy, Alkoxycarbonyl, Alkenyloxycarbonyl, Alkanoyloxy, Alkenoyloxy, Halogen, Cyano, -NCS, -CF₃ oder -OCF₃ bedeutet.

12. Flüssigkristallines Gemisch mit mindestens 2 Komponenten, dadurch gekennzeichnet, dass mindestens eine Komponente eine Verbindung der in Anspruch 1 definierten Formel I ist.

13. Verwendung der Verbindungen der in Anspruch 1 definierten Formel I für elektro-optische Zwecke.

## Claims

1. Compounds of the general formula 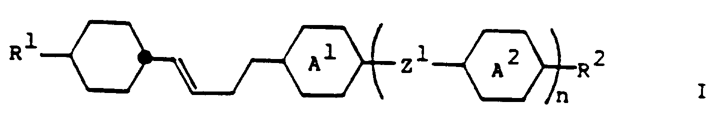 wherein n stands for the number 0 or 1; R¹ denotes a group R³ or R³-A³-Z²- and R² denotes a group R⁴ or R⁴-A⁴-Z³-; ring A¹ signifies unsubstituted or halogen-, cyano- and/or methyl-substituted 1,4-phenylene in which optionally 1 or 2 CH groups is/are replaced by nitrogen; A³, A⁴ and ring A² each independently represent unsubstituted or halogen-, cyano- and/or methyl-substituted 1,4-phenylene in which optionally 1 or 2 CH groups is/are replaced by nitrogen or trans-1,4-cyclohexylene, trans-1,3-dioxane-2,5-diyl, 1-cyano-trans-1,4-cyclohexylene, bicyclo[2.2.2]octane-1,4-diyl, naphthalene-2,6-diyl, tetralin-2,6-diyl or transdecalin-2,6-diyl; Z¹, Z² and Z³ each independently signify a single covalent bond, -COO-, -OOC-, -CH₂O-, -OCH₂-, -CH₂CH₂-, -C≡C-, -(CH₂)₃O-, -O(CH₂)₃-, -(CH₂)₄- or the trans form of -CH=CH-CH₂CH₂-, -CH₂CH₂-CH=CH-, -CH=CH-CH₂O- or -OCH₂-CH=CH-; R³ and R⁴ each independently denote halogen, cyano, -NCS, -CF₃, -OCF₃ or alkyl in which optionally one >CH-CH< is replaced by >C=C< and/or optionally one methylene group or two non-adjacent methylene groups is/are replaced by -O-, -COO- and/or -OOC- and/or optionally one methylene group is replaced by -CHX-; and X signifies halogen, cyano or methyl, with the exception of 1,2,6-trifluoro-4-[4E-(trans-4-pentylcyclohexyl)-3-butenyl]benzene.

2. Compounds according to claim 1, characterized by the general formula 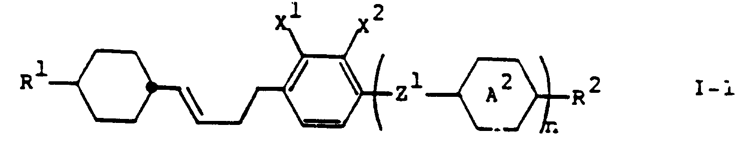 wherein n, R¹, R², Z¹ and ring A² have the significances given in claim 1 and X¹ and X² each independently denote hydrogen, halogen, cyano or methyl.

3. Compounds according to claim 1 or 2, characterized in that A³ and A⁴ each independently represent trans-1,4-cyclohexylene or unsubstituted or halogen-, cyano- and/or methyl-substituted 1,4-phenylene.

4. Compounds according to any one of claims 1 to 3, characterized in that ring A² stands for unsubstituted 1,4-phenylene or 1,4-phenylene monosubstituted or 2,3-disubstituted with halogen, cyano and/or methyl, for trans-1,4-cyclohexylene, trans-1,3-dioxane-2,5-diyl, 1-cyano-trans-1,4-cyclohexylene, bicyclo[2.2.2]octane-1,4-diyl, naphthalene-2,6-diyl, tetralin-2,6-diyl, trans-decalin-2,6-diyl, pyridine-2,5-diyl, pyrimidine-2,5-diyl or pyrazine-2,5-diyl.

5. Compounds according to any one of claims 1 to 4, characterized in that Z¹, Z² and Z³ each signify a single covalent bond or one of the groups Z¹, Z² and Z³ also signifies -COO-, -OOC-, -CH₂O-, -OCH₂-, -CH₂CH₂-, -C≡C-, -(CH₂)₃O-, -O(CH₂)₃-, -(CH₂)₄- or the trans form of -CH=CH-CH₂CH₂-, -CH₂CH₂-CH=CH-, -CH=CH-CH₂O- or -OCH₂-CH=CH-.

6. Compounds according to any one of claims 1 to 5, characterized by the general formulae 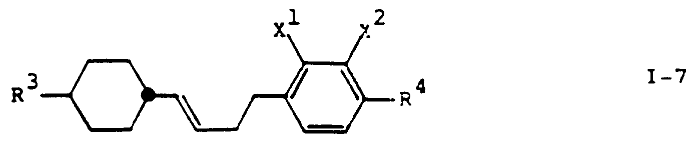 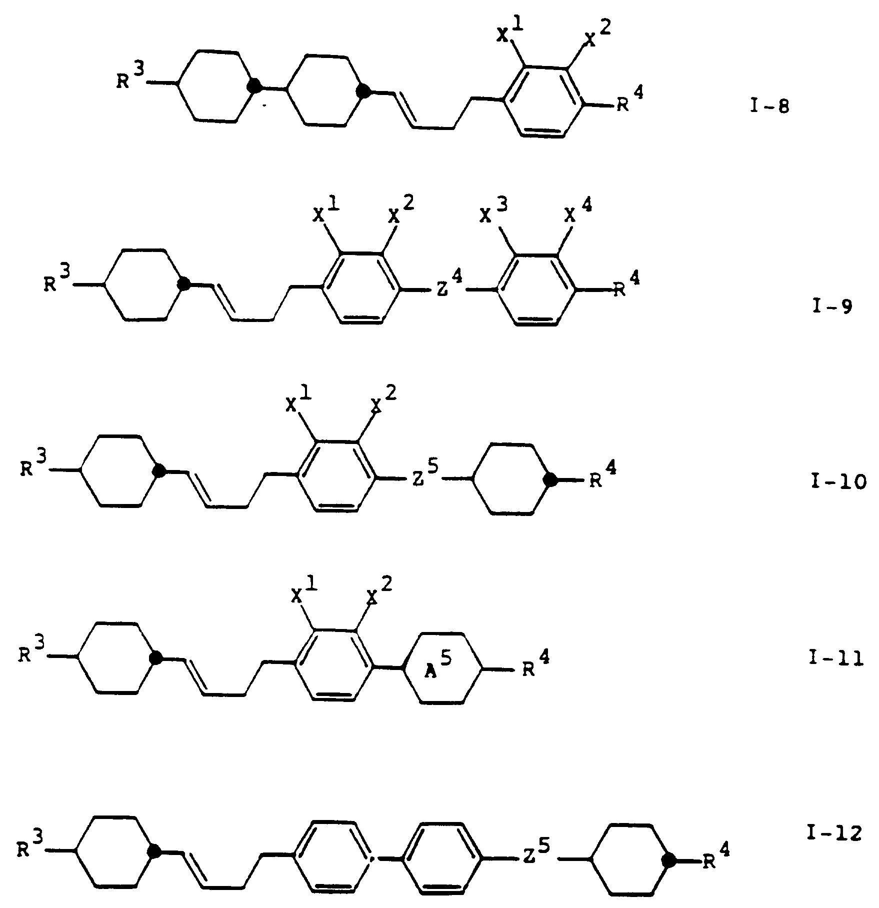 wherein R³, and R⁴ have the significances given in claim 1, X¹, X², X³ and X⁴ each independently denote hydrogen, halogen, cyano or methyl, Z⁴ signifies a single covalent bond, -COO-, -OOC- or -C≡C-; Z⁵ denotes a single covalent bond, -OOC-, -OCH₂-, -CH₂CH₂-, -C≡C-, -O(CH₂)₃-, -(CH₂)₄- or the trans form of -CH₂CH₂-CH=CH- or OCH₂-CH=CH-; and ring A⁵ represents pyridine-2,5-diyl, pyrimidine-2,5-diyl, pyrazine-2,5-diyl, trans-1,3-dioxane-2,5-diyl, bicyclo[2.2.2]octane-1,4-diyl, naphthalene-2,6-diyl, tetralin-2,6-diyl or trans-decalin-2,6-diyl.

7. Compounds according to claim 2 or 6, characterized in that X¹, X², X³ and X⁴ each independently signify hydrogen or fluorine.

8. Compounds according to any one of claims 1 to 7, characterized in that R³ and R⁴ each independently denote alkyl in which optionally one >CH-CH< is replaced by >C=C< and/or optionally one methylene group or two non-adjacent methylene groups is/are replaced by -O-, -COO- and/or -OOC- and/or optionally one methylene group is replaced by -CHX- or R⁴ also denotes halogen, cyano, -NCS, -CF₃ or -OCF₃.

9. Compounds according to any one of claims 1 to 8, characterized in that R³ and R⁴ each have a maximum of 18, preferably a maximum of 12, carbon atoms.

10. Compounds according to any one of claims 1 to 9, characterized in that R³ signifies alkyl, alkenyl, alkoxy, alkenyl-oxy, alkoxycarbonyl, alkenyloxycarbonyl, alkanoyloxy or alkenoyloxy, preferably alkyl or alkenyl.

11. Compounds according to any one of claims 1 to 10, characterized in that R⁴ signifies alkyl, alkenyl, alkoxy, alkenyloxy, alkoxycarbonyl, alkenyloxycarbonyl, alkanoyloxy, alkenoyloxy, halogen, cyano, -NCS, -CF₃ or -OCF₃.

12. A liquid crystalline mixture with at least 2 components, characterized in that at least one component is a compound of formula I defined in claim 1.

13. The use of the compounds of formula I defined in claim 1 for electro-optical purposes.

## Revendications

1. Composés de formule 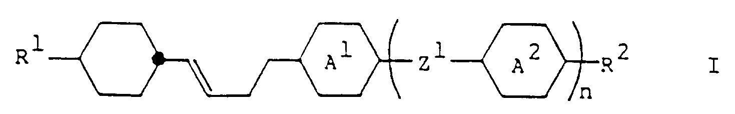 dans laquelle n est le nombre 0 ou 1; R¹ est un groupe R³ ou R³-A³-Z²- et R² un groupe R⁴ ou R⁴-A⁴-Z³-; le cycle A¹ représente un 1,4-phénylène non substitué ou substitué par un halogène, cyano et/ou méthyle, dans lequel le cas échéant 1 ou 2 groupes CH sont remplacés par l'azote; A³, A⁴ et le cycle A² représentent indépendamment l'un de l'autre un 1-4-phénylène, dans lequel le cas échéant 1 ou 2 groupes-CH- sont remplacés par l'azote, non substitué ou substitué par un halogène, cyano et/ou méthyle, ou les trans-1,4-cyclohexylène, trans-1,3-dioxane-2-5-diyle, 1-cyano-trans-1,4-cyclohexylène, bicyclo[2.2.2]octane-1,4-diyle, naphtalène-2,6-diyle, tétralin-2,6-diyle ou transdécalin-2,6-diyle; Z¹, Z² et Z³ représentent indépendamment l'un de l'autre une liaison de covalence simple, -COO-, -OOC-, -CH₂O-, -OCH₂, -CH₂CH₂-, -C≡C-, -(CH₂)₃O-, -O(CH₂)₃-, -(CH₂)₄- ou la forme trans de -CH=CH-CH₂CH₂-, -CH₂CH₂-CH=CH-, -CH=CH-CH₂O- ou -OCH₂-CH=CH-; R³ et R⁴ représentent indépendamment l'un de l'autre un halogène, cyano, -NCS, -CF₃, -OCF₃ ou un alkyle, dans lequel, le cas échéant un groupe >CH-CH< est remplacé par >C=C< et/ou le cas échéant un ou deux groupes méthylènes non voisins sont remplacés par -0-, -COO- et/ou -OOC- et/ou le cas échéant un groupe méthylène est remplacé par -CHX-; et X représente un halogène, cyano ou méthyle, à l'exception du 1,2,6-trifluoro-4-[4E-(trans-4-pentylcyclohexyl)-3-butényl]benzène.

2. Composés selon la revendication 1, caractérisés par la formule générale 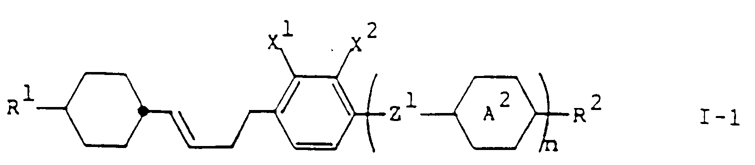 dans laquelle n, R¹, R², Z¹ et le cycle A² ont les significations données à la revendication 1 et X¹ et X² indépendamment l'un de l'autre représentent un hydrogène, halogène, cyano ou méthyle.

3. Composés selon la revendication 1 ou 2, caractérisés en ce que A³ et A⁴ représentent indépendamment l'un de l'autre un trans-1,4-cyclohexylène ou un 1,4-phénylène non substitué ou substitué par un halogène, cyano et/ou méthyle.

4. Composés selon l'une des revendications 1 à 3, caractérisés en ce que le cycle A² représente un 1,4-phénylène non substitué ou monosubstitué ou 2,3-disubstitué par un halogène, cyano et/ou méthyle, ou représente un trans-1,4-cyclohexylène, trans-1,3-dioxane-2,5-diyle, 1-cyano-trans-1,4-cyclohexylène, bicyclo [2.2.2]octane-1,4-diyle, naphtalène-2,6-diyle tétralin-2,6-diyle, trans-décalin-2,6-diyle, pyridine-2,5-diyle, pyrimidine-2,5-diyle ou pyrazine-2,5-diyle.

5. Composés selon l'une des revendications 1 à 4, caractérisés en ce que Z¹, Z² et Z³ représentent chacun une liaison simple de covalence ou l'un des groupes Z¹, Z² et Z³ représente aussi -COO-, -OOC, -CH₂O-, -OCH₂-, -CH₂CH₂-, -C≡C-, -(CH₂)₃O-, -O(CH₂)₃-, ((CH₂)₄- ou la forme trans de -CH=CH-CH₂-CH₂-, -CH₂CH₂-CH=CH-, -CH=CH-CH₂O ou -OCH₂-CH=CH-.

6. Composés selon l'une des revendications 1 à 5, caractérisés par les formules générales: 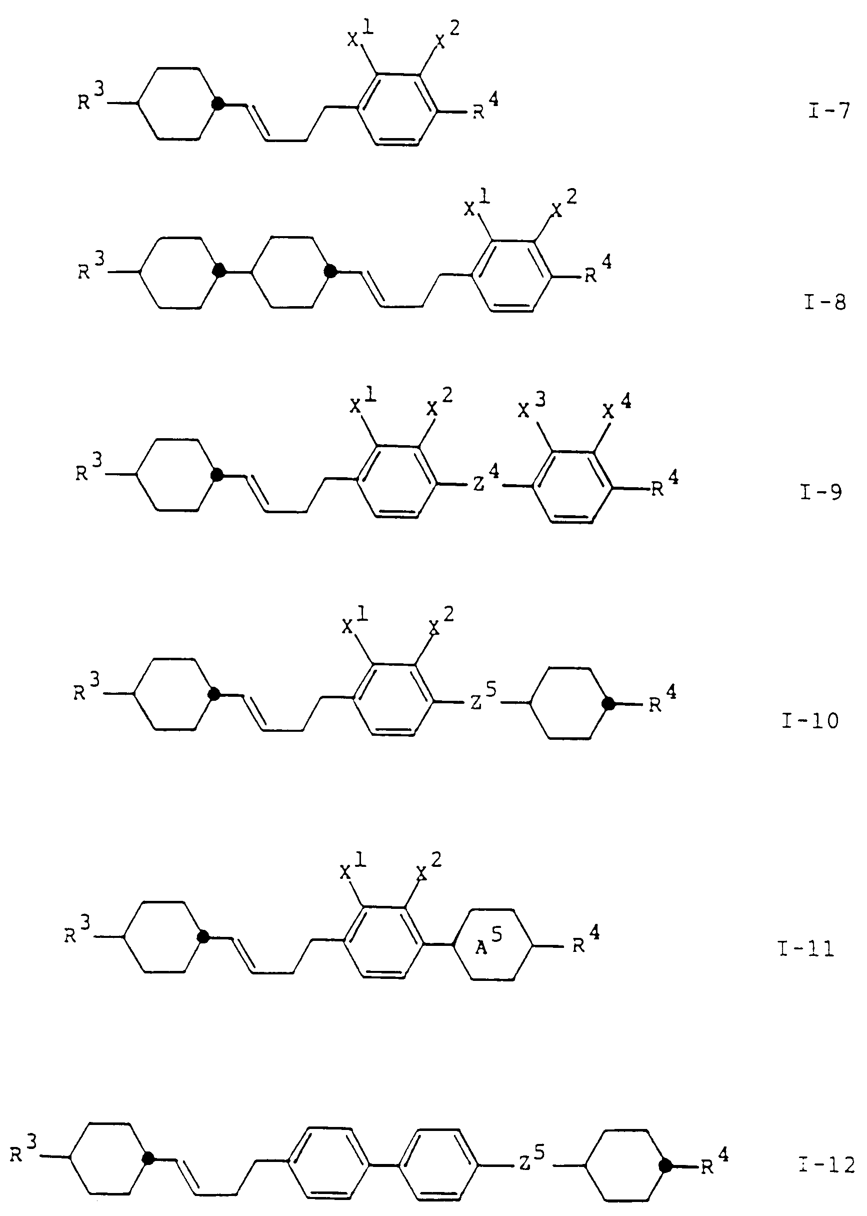 dans lesquelles R³ et R⁴ ont les significations données ci-dessus ; X¹, X², X³ et X⁴ représentent indépendamment l'un de l'autre un hydrogène, halogène, cyano ou méthyle; Z⁴ est une simple liaison de covalence, -COO-, -OOC-ou -C≡C-; Z⁵ est une simple liaison de covalence,-OOC-, -OCH₂-, -CH₂CH₂-, -C≡C-, -O(CH₂)₃-, -(CH₂)₄- ou la forme trans de -CH₂CH₂-CH=CH- ou -OCH₂-CH=CH-, et le cycle A⁵ est pyridine-2,5-diyle, pyrimidine-2,5-diyle, pyrazine-2,5-diyle, trans-1,3-dioxane-2,5-diyle, bicyclo[2.2.2] octane-1,4-diyle, naphtalène-2,6-diyle, tétralin-2,6-diyle ou trans-décalin-2,6-diyle.

7. Composés selon la revendication 2 ou 6, caractérisés en ce que X¹, X², X³ et X⁴ représentent indépendamment l'un de l'autre l'hydrogène ou le fluor.

8. Composés selon l'une des revendications 1 à 7, caractérisés en ce que R³ et R⁴ indépendamment l'un de l'autre représentent un alkyle, dans lequel le cas échéant un >CH-CH< est remplacé par >C=C< et/ou le cas échéant un ou deux groupes méthylènes non voisins sont remplacés par -O-, -COO- et/ou -OOC- et/ou le cas échéant un groupe méthylène est remplacé par -CHX-, ou R⁴ représente aussi un halogène, cyano, -NCS, CF₃ ou -OCF₃.

9. Composés selon l'une des revendications 1 à 8, caractérisés en ce que R³ et R⁴ présentent au plus chacun 18, de préférence au plus chacun 12 atomes de carbone.

10. Composés selon l'une des revendications 1 à 9, caractérisés en ce que R³ représente un alkyle, alcényle, alcoxy, alcényloxy, alcoxycarbonyle, alcényloxycarbonyle, alcanoyloxy ou alcénoyloxy, de préférence alkyle ou alcényle.

11. Composés selon l'une des revendications 1 à 10, caractérisés en ce que R⁴ représente un alkyle, alcényle, alcoxy, alcényloxy, alcoxycarbonyle, alcényloxycarbonyle, alcanoyloxy, alcénoyloxy, halogène, cyano, -NCS, -CF₃ ou -OCF₃.

12. Mélange de cristaux liquides avec au moins deux composants, caractérisé en ce qu'au moins un composant est un composé de la formule I définie à la revendication 1.

13. Utilisation de composés de formule I définie à la revendication 1 pour des utilisations électro-optiques.
